# EUROPEAN PATENT SPECIFICATION

(11) **EP 4 199 896 B1**
(45) Date of publication and mention of the grant of the patent: **21.01.2026**
(21) Application number: 22719157.4
(22) Date of filing: 06.04.2022
(51) Int. Cl.: A61K 8/46, A61K 8/44, A61Q 19/10, A61K 8/37, A61K 8/04

(54) **LIQUID PERSONAL CARE COMPOSITIONS AND METHODS FOR THE SAME**
FLÜSSIGE KÖRPERPFLEGEZUSAMMENSETZUNGEN UND VERFAHREN DAFÜR
COMPOSITIONS DE SOINS PERSONNELS LIQUIDES ET PROCÉDÉS ASSOCIÉES

(30) Priority: 09.04.2021 US 202163201040 P
(43) Date of publication of application: 28.06.2023
(73) Proprietor: Colgate-Palmolive Company, New York, NY 10022 (US)
(72) Inventor: NWOSISI, Juliana, Plainfield, New Jersey 07080 (US); LESNIAK, Ewelina, Linden, New Jersey 07036 (US)
(74) Representative: Wichmann, Hendrik
(86) International application number: PCT/US2022/023619
(87) International publication number: WO 2022/216791

(56) References cited:
- EP-B1- 1 771 538
- WO-A1-2020/182318
- AU-A1- 2017 204 070
- US-A1- 2013 090 279
- US-B2- 6 770 607

## Description

This application claims the benefit of priority from U.S. Provisional Application No. 63/201,040, filed April 9, 2021.

### BACKGROUND

Liquid personal care compositions require minimal levels of active ingredients, such as surfactants, to provide sufficient properties related to consumer perception (e.g., sensory properties), such as cleaning efficacy and foaming. The amount or concentration of the active ingredient necessary to provide these properties in the liquid personal care compositions, however, is often cost prohibitive. For example, high levels of surfactants, such as sodium laureth sulfate (SLES) and cocamidopropyl betaine (CAPB), are costly; and thus, there is a desire to minimize their use in the liquid personal care compositions to reduce the cost of production and maintain a low cost product for consumers. While reducing the concentration of surfactants utilized in the liquid personal care compositions may be desirable from a cost perspective, reducing the concentration of surfactants may negatively affect other properties of the liquid personal care compositions.

What is needed, then, are improved, cost effective liquid personal care compositions having a reduced amount of active ingredients that provides comparable or superior properties to conventional liquid personal care compositions. AU 2017204070 A1 discloses personal care compositions containing complexing polyelectrolytes. US 6,770,607 B2 discloses a viscoelastic cleansing gel composition. US 2013/0090279 A1 discloses a shampoo composition having a combination of sodium lauryl sulfate and sodium laureth-n sulfate. WO 2020/182318 A1 discloses an antimicrobial mixture comprising or consisting of monocyclic sesquiterpene alcohol, at least one active selected from 4-hydroxy acetophenone, sorbitan caprylate, cocamidopropyl PG-dimonium chloride phosphate, tetrasodium glutamate diacetate, glyceryl caprylate and glyceryl caprate, and optionally (c) at least one additional antimicrobial agent different from components (a) and (b).

### BRIEF SUMMARY

This summary is intended merely to introduce a simplified summary of some aspects of one or more implementations of the present disclosure. Further areas of applicability of the present disclosure will become apparent from the detailed description provided hereinafter. This summary is not an extensive overview. Rather, its purpose is merely to present one or more concepts in simplified form as a prelude to the detailed description below.

The foregoing and/or other aspects and utilities embodied in the present disclosure are achieved by providing a personal care composition including a surfactant system and a thickener as further defined in the claims.

The surfactant system includes at least one anionic surfactant and at least one a zwitterionic surfactant as defined in the claims.

The anionic surfactant includes sodium laureth sulfate (SLES) as defined in the claims, optionally further ammonium lauryl ether sulfate, sodium lauryl sulfate, ammonium lauryl sulfate, triethanolamine lauryl sulfate, disodium laureth sulfosuccinate, sodium cocoyl isethionate, sodium C12-C14 olefin sulfonate, sodium laureth-6 carboxylate, sodium C12-C15 pareth sulfate, sodium methyl cocoyl taurate, sodium dodecylbenzene sulfonate, sodium cocoyl sarcosinate, triethanolamine monolauryl phosphate, or combinations thereof.

The zwitterionic surfactant includes one or more of cocamidopropyl betaine and lauryl hydroxysultaine as defined in the claims, optionally further one or more of a betaine, a sultaine, an amino propionate, or combinations thereof.

In at least one implementation, the betaine further includes one or more of imidazoline-based betaines, alkyl dimethyl aminoacetic acid betaines, sulfobetaines, or combinations thereof.

In at least one implementation, the zwitterionic surfactant includes at least one betaine and excludes sultaines.

In at least one implementation, the surfactant system is present in an amount of from 6.8 wt% to about 8.5 wt%, more preferably about 7 wt% to about 7.5 wt%, based on the total weight of the personal care composition.

The surfactant system includes at least one anionic surfactant and at least one zwitterionic surfactant as defined in the claims; wherein the at least one anionic surfactant includes sodium laureth sulfate, wherein the at least one zwitterionic surfactant includes one or more of cocamidopropyl betaine, lauryl hydroxysultaine, or combinations thereof, wherein the sodium laureth sulfate is present in an amount from 4 wt% to less than 7.8 wt% as defined in the claims, optionally about 4.5 wt% to about 6.5 wt%, preferably, about 5 wt% to about 5 wt%, more preferably, about 5.6 wt%, based on the total weight of the personal care composition, wherein the cocamidopropyl betaine is present in an amount of from 0.5 wt% to 2.0 wt% as defined in the claims, optionally about 0.6 wt% to about 1.8 wt%, about 0.8 wt% to about 1.6 wt%, preferably about 1.0 wt% to about 1.4 wt%, or more preferably about 1.2 wt%, and wherein the lauryl hydroxysultaine, if present, is present in an amount of from about 0.2 wt% to about 1.0 wt% as defined in the claims, preferably about 0.3 wt% to about 0.9 wt%, more preferably about 0.4 wt% to about 0.8 wt%, even more preferably about 0.5 wt% to about 0.7 wt%, or about 0.6 wt%, based on the total weight of the personal care composition.

The personal care composition comprises less than 0.05 weight% polyquaternium, as defined in the claims, optionally free of cocomonoethanolamide (CMEA), or combination thereof.

The thickener includes esters of polyalkoxylated polyols and fatty acids as defined in the claims, preferably a tetra ester made of fatty acids and a polyoxyethylene pentaerythritol having four hydrophilic poly-(ethylene glycol) arms, even more preferably, a PEG-150 pentaerythrityl tetrastearate having four hydrophilic poly-(ethelene glycol) arms capped with stearic fatty acids.

The thickener is present in an amount of from about 0.08 wt% to about 0.12 wt% as defined in the claims, preferably, about 0.09 wt% to about 0.11 wt%, more preferably, about 0.10 wt%, based on the total weight of the personal care composition.

In at least one implementation, the surfactant system and the thickener are present in an amount of from about 6.8 wt% to less than 10 wt%, preferably about 6.8 wt% to about 8.5 wt%, more preferably about 7 wt% to about 7.5 wt%, based on the total weight of the personal care composition.

In at least one implementation, a weight ratio of the anionic surfactant to the thickener is from about 45:1 to about 70:1, about 50:1 to about 65:1, preferably about 55:1 to about 60:1, more preferably about 56:1 to about 57:1.

In at least one implementation, a weight ratio of the betaine to the thickener is from about 9.5:1 to about 16:1, more preferably about 10:1 to about 15:1, even more preferably about 12:1 to about 14:1, even more preferably about 12:1.

In at least one implementation, the personal care composition includes a salt present in an amount of about 1.25 wt% or less, based on the total weight of the personal care composition.

In at least one implementation, the personal care composition includes a viscosity of from about 4,000 mPa·s (cP) to about 7,500 mPa·s (cP), about 4,500 mPa·s (cP) to about 7,000 mPa·s (cP), or about 5,000 mPa·s (cP) to about 6,500 mPa·s (cP), at about 25°C.

In at least one implementation, the personal care composition includes a pH of from about 4.0 to about 6.5, about 4.5 to about 6, more preferably, about 4.5 to about 5.4, about 4.8 to about 5.4, or about 5.

In at least one implementation, the surfactant system and the thickener are present in a synergistic amount to provide comparable foam volume, reduced stringiness, increased structure, and increased fragrance release as compared to conventional personal care compositions, wherein the conventional personal care compositions comprise a surfactant system and a thickener in an amount of about 10 wt% or greater, based on the total weight of the conventional personal care composition.

In at least one implementation, the personal care composition maintains a pH of about 4.9 to about 5.3, about 5.0 to about 5.2, or about 5.1, after exposure to accelerated aging conditions.

In at least one implementation, the personal care composition maintains a viscosity of about 4,000 mPa·s (cP) to about 7,500 mPa·s (cP), about 4,500 mPa·s (cP) to about 7,000 mPa·s (cP), or about 5,000 mPa·s (cP) to about 6,500 mPa·s (cP), when exposed to accelerated aging conditions.

The foregoing and/or other aspects and utilities embodied in the present disclosure may be achieved by providing a method for preparing the personal care composition disclosed herein. The method including contacting the surfactant system and the thickener with one another.

Further areas of applicability of the present disclosure will become apparent from the detailed description provided hereinafter. It should be understood that the detailed description and specific examples, while indicating some typical aspects of the disclosure, are intended for purposes of illustration only and are not intended to limit the scope of the disclosure which is defined by the appended claims.

### BRIEF DESCRIPTION OF THE DRAWINGS

The present disclosure is best understood from the following detailed description when read with the accompanying Figures. It is emphasized that, in accordance with the standard practice in the industry, various features are not drawn to scale. In fact, the dimensions of the various features may be arbitrarily increased or reduced for clarity of discussion.
Figure 1 illustrates a plot or graphical representation of the foam volume for Example 4.
Figure 2 illustrates a plot or graphical representation of the stringiness for Example 4.
Figure 3 illustrates a plot or graphical representation of the structure of the personal care compositions of Example 4.

### DETAILED DESCRIPTION

The following description of various typical aspect(s) is merely exemplary in nature and is in no way intended to limit the disclosure, which is defined by the appended claims.

As used throughout this disclosure, ranges are used as shorthand for describing each and every value that is within the range.

Unless otherwise specified, all percentages and amounts expressed herein and elsewhere in the specification should be understood to refer to percentages by weight. The amounts given are based on the active weight of the material.

Additionally, all numerical values are "about" or "approximately" the indicated value, and take into account experimental error and variations that would be expected by a person having ordinary skill in the art. It should be appreciated that all numerical values and ranges disclosed herein are approximate values and ranges, whether "about" is used in conjunction therewith. It should also be appreciated that the term "about," as used herein, in conjunction with a numeral refers to a value that may be ± 0.01% (inclusive), ± 0.1% (inclusive), ± 0.5% (inclusive), ± 1% (inclusive) of that numeral, ± 2% (inclusive) of that numeral, ± 3% (inclusive) of that numeral, ± 5% (inclusive) of that numeral, ± 10% (inclusive) of that numeral, or ± 15% (inclusive) of that numeral.

As used herein, "free" or "substantially free" of a material may refer to a composition, component, or phase where the material is present in an amount of less than 1.0 weight %, less than 0.1 weight %, less than 0.05 weight %, less than 0.01 weight %, less than 0.005 weight %, or less than 0.0001 weight % based on a total weight of the composition, component, or phase.

In the event of a conflict in a definition in the present disclosure and that of a cited reference, the present disclosure controls.

The present inventors have surprisingly and unexpectedly discovered that personal care compositions including a surfactant system and a thickener, where the surfactant system and/or the thickener is present in an amount of less than 10 wt%, based on the total weight of the personal care composition, exhibited sensory properties, such as foam volume and/or foam stability, that were at least parity with respect to conventional personal care compositions including about or greater than 10 wt% of the surfactant system and/or thickener. More particularly, the present inventors have surprisingly and unexpectedly discovered that the surfactant system, including at least one anionic surfactant and at least one zwitterionic surfactant, interacts synergistically with the thickener to provide comparable foam volume, reduced stringiness, increased structure, and increased fragrance release as compared to conventional personal care compositions including about or greater than 10 wt% of the surfactant system and/or thickener.

The present inventors have also surprisingly and unexpectedly discovered that personal care compositions including a surfactant system and a thickener, where the surfactant system and/or the thickener is present in an amount of less than 10 wt%, based on the total weight of the personal care composition, exhibited relatively greater naturality and sustainability as compared to conventional personal care compositions including about or greater than 10 wt% of the surfactant system and/or thickener. More particularly, the present inventors have surprisingly and unexpectedly discovered that the surfactant system, including at least one anionic surfactant and at least one zwitterionic surfactant, interacts synergistically with the thickener to provide increased naturality and sustainability as compared to conventional personal care compositions including about or greater than 10 wt% of the surfactant system and/or thickener.

The present inventors have also surprisingly and unexpectedly discovered that stable personal care compositions may be prepared by combining a surfactant system and a thickener, where the surfactant system and/or the thickener is present in an amount of less than 10 wt%, based on the total weight of the personal care composition. Particularly, the present inventors have surprisingly and unexpectedly discovered that the surfactant system, including at least one anionic surfactant and at least one zwitterionic surfactant, interacts synergistically with the thickener to provide stable personal care compositions having a pH of about 4.9 to about 5.3, about 5.0 to about 5.2, or about 5.1, and/or a viscosity of about 4,000 mPa·s (cP) or greater, about 5,000 mPa·s (cP) or greater, about 6,000 mPa·s (cP) or greater, or about 7,000 mPa·s (cP) or greater. The present inventors have also surprisingly and unexpectedly discovered that the surfactant system, including at least one anionic surfactant and at least one zwitterionic surfactant, interacts synergistically with the thickener to provide stable personal care compositions having a pH of about 4.9 to about 5.3, about 5.0 to about 5.2, or about 5.1, and/or a viscosity of about 4,000 mPa·s (cP) or greater, about 5,000 mPa·s (cP) or greater, about 6,000 mPa·s (cP) or greater, or about 7,000 mPa·s (cP) or greater, when exposed to accelerated aging conditions.

### COMPOSITIONS

Compositions disclosed herein are personal care compositions. For example, compositions disclosed herein may be a personal care composition, a personal care product, or form a portion of the personal care composition or the personal care product. Illustrative personal care products or compositions thereof may be or include, but are not limited to, body washes, shower gels, liquid soaps (e.g., liquid hand soaps), face washes, shampoos, hair conditioners, or the like. In a preferred implementation, the personal care product or the composition thereof is a liquid personal care composition, such as a shower gel, a liquid hand soap, a body wash, or the like.

The compositions disclosed herein are personal care composition including one or more of a surfactant system, a thickener, or combinations thereof, where the surfactant system and/or the thickener are present in an amount of less than or equal to about 10 wt%, based on the total weight of the personal care composition as further defined in the claims. As further described herein, the surfactant system includes one or more anionic surfactants, one or more amphoteric or zwitterionic surfactants or combinations thereof, and the thickener includes one or more esters of polyalkoxylated polyols and fatty acids as defined in the claims. The surfactant system and/or the thickener may also be capable of or configured to facilitate or provide stability to the personal care composition, such as under accelerated aging conditions. The surfactant system and/or the thickener may also be capable of or configured to provide enhanced or comparable sensory properties as compared to conventional personal care compositions. As used herein, the term or expression "conventional personal care composition" may refer to a composition including actives, such as a surfactant system and/or a thickener, in an amount of about 9 wt% or greater, about 9.5 wt% or greater, about 10 wt% or greater, about 10.5 wt% or greater, or about 11 wt% or greater, based on the total weight of the conventional personal care composition.

The personal care compositions disclosed herein include a surfactant system and/or a thickener in an amount of less than 10 wt% and may exhibit comparable, enhanced, or relatively greater stability as compared to conventional personal care compositions, based on the total weight of the personal care composition. For example, the personal care compositions disclosed herein may exhibit comparable, enhanced, or relatively greater stability as compared to conventional personal care compositions including a surfactant system and/or thickener, where the surfactant system of the conventional personal care compositions includes one or more of sodium laureth sulfate, cocamidopropyl betaine, cocomonoethanolamide, or combinations thereof, and wherein the surfactant system and/or thickener of the conventional personal care composition may be present in an amount of about 10 wt% or greater, based on the total weight of the conventional personal care composition. In another example, the personal care compositions disclosed herein may exhibit no phase separation, relatively stable pH, and/or relatively stable viscosity when exposed to various accelerated aging conditions as compared to conventional personal care compositions including a surfactant system and/or a thickener, where the surfactant system of the conventional personal care compositions includes one or more of sodium laureth sulfate, cocamidopropyl betaine, cocomonoethanolamide, or combinations thereof, and wherein the surfactant system and/or the thickener of the conventional personal care compositions are present in an amount of about 10 wt% or greater, based on the total weight of the conventional personal care composition. In another example, the personal care compositions disclosed herein may exhibit no phase separation, may maintain a clear appearance that does not become hazy with time or temperature change or addition of other formula ingredients, a relatively stable pH of about 3.5 to about 6.0, about 4.0 to about 5.4, about 4.9 to about 5.3, about 5.0 to about 5.2, or about 5.1, and/or a relatively stable viscosity of about 4,000 mPa·s (cP) or greater, or about 5,000 mPa·s (cP) or greater, when exposed to various accelerated aging conditions as compared to conventional personal care compositions.

As used herein, the term or expression "relatively stable" may refer to a property or value that does not change (e.g., increase or decrease) from an original property or value by an amount greater than 0.5%, greater than 1%, greater than 1.5%, greater than 2%, greater than 2.5%, greater than 3%, greater than 3.5%, greater than 4%, greater than 4.5%, greater than 5%, greater than 6%, greater than 7%, greater than 8%, greater than 9%, or greater than 10%. As further described herein, the personal care composition disclosed herein may include a surfactant system and a thickener capable of or configured to provide stability to the personal care composition with respect to pH and/or viscosity. Further, the personal care compositions disclosed herein provide comparable or enhanced stability as compared to conventional personal care compositions.

The personal care compositions disclosed herein, which include the surfactant system and the thickener in an amount of less than 10 wt% as defined in the claims, may exhibit comparable (e.g., parity) or enhanced sensory properties as compared to conventional personal care compositions including a surfactant system present in an amount of from about 10 wt% or greater, based on the total weight of the personal care composition. For example, the personal care compositions disclosed herein may exhibit comparable or enhanced sensory properties as compared to conventional personal care compositions including a surfactant system and/or a thickener, where the surfactant system includes one or more of sodium laureth sulfate, cocamidopropyl betaine, cocomonoethanolamide, or combinations thereof, and wherein the surfactant system and/or the thickener are present in an amount of about 10 wt% or greater, based on the total weight of the conventional personal care composition. For example, the personal care compositions disclosed herein may exhibit comparable or enhanced sensory properties as compared to conventional personal care compositions including a surfactant system, where the surfactant system includes one or more of sodium laureth sulfate, cocamidopropyl betaine, cocomonoethanolamide, or combinations thereof, and wherein the surfactant system and/or the thickener are present in an amount of about 10 wt% or greater, based on the total weight of the conventional personal care composition as further defined in the claim.

As discussed above, the personal care composition disclosed herein may exhibit comparable (e.g., parity) or enhanced sensory properties as compared to conventional personal care compositions. Illustrative sensory properties may be or include, but are not limited to, one or more of foam volume, foam thickness, foam stability, structure, stringiness, fragrance release, or combinations thereof.

As used herein, the term or expression "foam volume" may refer to a height or volume of foam above any non-foamed portion of a composition. Foam volume may be measured with a foam analyzer or foam tester, such as the SITA FoamTester R-200, which is commercially available from SITA Lab Solutions of Dresden, Germany.

As used herein, the term or expression "foam thickness" may refer to an amount of lather and/or a quality of the lather of a personal care composition as determined by a panel, such as an expert panel. The determination by the panel may be via a questionnaire comparing a first composition or a test composition and a second or control composition. The questionnaire may be scaled such as a scale from 1 to 10 or from 1-5.

As used herein, the term or expression "foam stability" may refer to a measure of a foam to self-suspend or maintain its form. Foam stability may be evaluated visually. For example, foam stability may be measured by wetting hands with water, dispensing a composition to be evaluated in the hands (e.g., about 3 mL), rubbing into the hands, adding water to generate foam, collecting the foam in the hands, and recording how long the foam maintains its shape and/or height, and/or recording how long the foam takes to collapse.

As used herein, the term or expression "structure" may refer to the ability of the personal care composition to suspend substances, such as particles, exfoliants, beads, oils, or the like. The structure of the personal care composition may refer to the rheological profile, which may include viscoelastic parameters, structural properties or dependence of the composition, temperature dependence of the composition, or the like, or combinations thereof. The viscoelastic parameters may be or include plateau modulus, relaxation time, zero shear viscosity, or the like, or combinations thereof. It should be appreciated that these parameters at least partially characterize micellar shape, size, and/or density, which are at least partially correlated to structure and/or bulk formula behavior. Bulk formula behavior may refer to both consumer usage, product manufacturability, or combinations thereof. The structural properties of the personal care composition may also be measured or calculated from rheometer data. Illustrative structural properties may be or include, but are not limited to, static yield stress and/or structural parameter.

As used herein, the term or expression "rheology" may refer to the study of the deformation and flow of matter or a composition.

As used herein, the term or expression "plateau modulus" may refer to a measure of the density of micelle packing in dyn/cm².

As used herein, the term or expression "yield stress" may refer to the stress required in order to induce movement in a fluid.

As used herein, the term or expression "structural parameter" may refer to a measure of how much structure a fluid or composition has. It should be appreciated that fluids or compositions with values less than one (1) have no structure, while fluids with values greater than one (1) have structure.

As used herein, the term or expression "stringiness" may refer to a rheological property of the composition, such as an ability of a micelle to alleviate an applied stress. For example, the stringiness of a composition may be determined via rheological experiments that measures the time it takes for micelles to alleviate an applied stress, which may be quantified as relaxation time (tₘ). It should be appreciated that the relaxation time is directly proportional to a stringiness of a composition. As used herein, relaxation time may refer to the time it takes for micelles to alleviate an applied stress, and may be measured in seconds.

As used herein, the term or expression "fragrance release" may refer to a measure of fragrance generated by a composition. Fragrance release may be measured directly with instrumentation, such as with a flame ionization detection (FID) gas chromatography (GC) or GC-FID. Measuring the fragrance release may be via analyzing respective headspace of the composition as a neat composition and/or as a dilute solution of the composition (e.g., 3% solution). The fragrance release may also be determined via an expert panel and/or a consumer panel. Determining fragrance release via the expert panel and/or the consumer panel may include instructing the panel to smell and/or evaluate the fragrance directly from a package, rating the fragrance based on a scale, and/or completing a questionnaire. The smelling and/or evaluation of the fragrance release by the panels may be determined during a predetermined usage period.

The personal care composition may have a viscosity, as determined using a viscometer with spindle 5, at 20 RPM, and at a temperature of about 25°C, of from about 3,000 mPa·s (cP) to about 9,000 mPa·s (centipoise (cP)). As used herein, the term "viscosity" may refer to the internal resistance to flow exhibited by a fluid or the ratio of shearing stress to rate of shear, and may be measured in poise or centipoises (cP). For example, the personal care composition disclosed herein may have a viscosity of from about 3,000 mPa·s (cP), about 3,200 mPa·s (cP), about 3,500 mPa·s (cP), about 3,800 mPa·s (cP), about 4,000 mPa·s (cP), about 4,200 mPa·s (cP), about 4,400 mPa·s (cP), about 4,600 mPa·s (cP), about 4,800 mPa·s (cP), about 5,000 mPa·s (cP), about 5,200 mPa·s (cP), about 5,400 mPa·s (cP), about 5,600 mPa·s (cP), about 5,800 mPa·s (cP), or about 6,000 mPa·s (cP) to about 6,200 mPa·s (cP), about 6,400 mPa·s (cP), about 6,600 mPa·s (cP), about 6,800 mPa·s (cP), about 7,000 mPa·s (cP), about 7,200 mPa·s (cP), about 7,400 mPa·s (cP), about 7,600 mPa·s (cP), about 7,800 mPa·s (cP), about 8,000 mPa·s (cP), about 9,000 mPa·s (cP), or greater. In another implementation, the personal care composition may have a viscosity of from about 3,000 mPa·s (cP) or greater to about 9,000 mPa·s (cP). For example, the personal care composition may have a viscosity of from about 3,000 mPa·s (cP) or greater, about 3,500 mPa·s (cP) or greater, about 4,000 mPa·s (cP) or greater, about 4,200 mPa·s (cP) or greater, about 4,400 mPa·s (cP) or greater, about 4,600 mPa·s (cP) or greater, about 4,800 mPa·s (cP) or greater, about 5,000 mPa·s (cP) or greater, about 5,200 mPa·s (cP) or greater, about 5,400 mPa·s (cP) or greater, about 5,600 mPa·s (cP) or greater, about 5,800 mPa·s (cP) or greater, about 6,000 mPa·s (cP) or greater, about 6,200 mPa·s (cP) or greater, about 6,400 mPa·s (cP) or greater, about 6,600 mPa·s (cP) or greater, or about 6,800 mPa·s (cP) or greater to about 9,000 mPa·s (cP). In a preferred implementation, the personal care composition has a viscosity of from about 4,000 mPa·s (cP) to about 7,500 mPa·s (cP), about 4,500 mPa·s (cP) to about 7,000 mPa·s (cP), or about 5,000 mPa·s (cP) to about 6,500 mPa·s (cP).

The personal care composition may have an acidic pH. For example, the personal care composition may have a pH of less than 7, less than 6, or more preferably less than or equal to about 5.4. In another example, the personal care composition may have a pH of from about 3.0, about 3.5, or about 4.0 to less than 7, less than 6.5, less than 6, less than 5.5, less than 5, or less than 4.5. In a preferred implementation, the personal care composition may have a pH of from about 4.0 to about 6.5, about 4.5 to about 6, more preferably about 4.5 to about 5.4, about 4.8 to about 5.4, or about 5.

The personal care composition may maintain a viscosity of from about 4,000 mPa·s (cP), about 4,500 mPa·s (cP), or about 5,000 mPa·s (cP) to about 6,000 mPa·s (cP), about 6,500 mPa·s (cP), about 7,000 mPa·s (cP), or about 7,500 mPa·s (cP), when exposed to accelerated aging conditions.

The personal care composition may maintain a pH of from about 3.0 to about 6.0, about 4.0 to about 5.5, about 4.8 to about 5.4, more preferably about 5.0 to about 5.2, or about 5.1, when exposed to accelerated aging conditions.

The personal care compositions disclosed herein may not exhibit any phase separation after exposure to accelerated aging conditions. The personal care compositions disclosed herein may not exhibit any phase separation after exposing the personal care composition to one or more freeze-thaw treatments. For example, the personal care composition disclosed herein may not exhibit any phase separation after freeze-thawing treatment between -30°C and room temperature for at least or about 3 cycles/treatments. In another example, the personal care composition disclosed herein may not exhibit any phase separation after freeze-thawing treatment between -10°C and room temperature for at least or about 3 cycles/treatments.

The personal care composition disclosed herein may not exhibit any phase separation after exposing the personal care composition to temperatures of about 25°C, about 40°C, or about 49°C, for extended periods of time. For example, the personal care composition disclosed herein may not exhibit any phase separation after maintaining the personal care composition at about 49°C for at least 4 weeks or more. In another example, the personal care composition disclosed herein may not exhibit any phase separation after maintaining the personal care composition at about 25°C for at least 4 weeks, at least 8 weeks, or at least 13 weeks, or more. In yet another example, the personal care composition disclosed herein may not exhibit any phase separation after maintaining the personal care composition at about 40°C for at least 4 weeks, at least 8 weeks, or at least 13 weeks, or more. It should be appreciated that phase separation in the personal care composition is determined visually.

The personal care composition disclosed herein may maintain a clear appearance that does not become hazy with time or temperature changes, or with the addition of other ingredients or components. In an exemplary implementation, the personal care compositions disclosed herein maintain a clear appearance when the actives, such as the surfactant system and/or the thickener, are present in an amount as low as about 6 wt%, about 6.5 wt%, about 7 wt%, or about 7.5 wt%, based on the total weight of the personal care composition. For example, the personal care composition disclosed herein may maintain a clear appearance when SLES is present in an amount of from about 4 wt% to about 5.5 wt% or about 4.5 wt% to about 5 wt%, CAPB is present in an amount of from about 0.5 wt% to about 1.5 wt% or about 0.8 wt% to about 1.3 wt%, a hydroxysultaine in an amount of about 0.1 wt% to about 1 wt% or about 0.25 wt% to about 0.75 wt%; a thickener is present in an amount of from 0.08 wt% to 0.12 wt% as defined in the claims. In another example, the personal care composition disclosed herein may maintain a clear appearance when SLES is present in an amount of about 5 wt%, CAPB is present in an amount of about 1.1 wt%, a hydroxysultaine in an amount of about 0.5 wt%, and/or a thickener in an amount of about 0.1 wt%. The clarity of the personal care composition may be at least partially defined by a turbidity thereof, which is measured as Nephelometric Turbidity Units (NTU). In at least one implementation, the personal care composition may maintain a turbidity of from about 3 NTU to about 40 NTU. For example, the personal care composition including the surfactant system and/or the thickener in an amount as low as about 6 wt%, about 6.5 wt%, about 7 wt%, or about 7.5 wt%, based on the total weight of the personal care composition, may maintain a turbidity of from about 3 NTU, about 5 NTU, about 10 NTU, about 15 NTU, or about 20 NTU to about 25 NTU, about 30 NTU, about 35 NTU, or about 40 NTU. In another example, the personal care composition including the surfactant system and/or the thickener in an amount as low as about 6 wt%, about 6.5 wt%, about 7 wt%, or about 7.5 wt%, based on the total weight of the personal care composition, may maintain a turbidity of from about 3 NTU or about 5 NTU to less than 40 NTU, less than 35 NTU, less than 30 NTU, or less than 25 NTU.

The surfactant system of the personal care composition includes at least one anionic surfactant and at least one zwitterionic surfactants as further defined in the claims. The one or more surfactants include, one or more anionic surfactants, one or more nonionic surfactants, one or more cationic surfactants, one or more amphoteric or zwitterionic surfactants, or combinations thereof as defined in the claims. The at least one amphoteric surfactant and the at least one anionic surfactant may be capable of or configured to interact synergistically with the thickener to provide sufficient or enhanced sensory properties to the personal care composition. In another example, the personal care composition including the combination of the amphoteric surfactant and the anionic surfactant may be capable of or configured to interact synergistically with the thickener to provide sufficient or enhanced stability to the personal care composition, such as during accelerated aging conditions.

The surfactant system includes at least one anionic surfactant as defined in the claims. The anionic surfactant comprises sodium lauryl ether sulfate or sodium laureth sulfate (SLES) and may further comprise ammonium lauryl ether sulfate, sodium lauryl sulfate, ammonium lauryl sulfate, triethanolamine lauryl sulfate, disodium laureth sulfosuccinate, sodium cocoyl isethionate, sodium C12-C14 olefin sulfonate, sodium laureth-6 carboxylate, sodium C12-C15 pareth sulfate, sodium methyl cocoyl taurate, sodium dodecylbenzene sulfonate, sodium cocoyl sarcosinate, triethanolamine monolauryl phosphate, or the like, or combinations thereof.

The surfactant system includes at least one zwitterionic surfactant as defined in the claims. As used herein, an amphoteric or zwitterionic surfactant may refer to a compound or surfactant that has the capacity of behaving either as an acid or a base or a compound or surfactant that carries both a positive and a negative charge. The amphoteric or zwitterionic surfactant comprises one or more of cocamidopropyl betaine and lauryl hydroxysultaine, optionally further betaines, sultaines, amino propionates or combinations thereof. In at least one implementation, the amphoteric surfactant includes at least the one or more betaines and excludes the sultaines. In a more preferred implementation, the amphoteric surfactant includes a combination of at least the one or more betaines and the one or more sultaines.

Illustrative further betaines may be or include, but are not limited to, one or more of imidazoline-based betaines, alkyl dimethyl aminoacetic acid betaines, fatty acid amide propyl betaines, sulfobetaines, or combinations thereof. In a preferred implementation, the further betaines may be or include one or more fatty acid amide propyl betaines or fatty acid amidopropyl betaines. Illustrative betaines may be or include, but are not limited to, lauryl betaine, cetyl betaine, coco betaine, lauramidopropyl betaine, coco-dimethylcarboxymethyl betaine, lauryldimethylcarboxymethyl betaine, lauryldimethylcarboxyethyl betaine, cetyldimethylcarboxymethyl betaine, lauryl-bis-(2-hydroxyethyl)carboxymethyl betaine, oleyldimethylgammacarboxypropyl betaine, lauryl-bis-(2-hydroxypropyl)-carboxyethyl betaine, or the like, or combinations thereof.

It should be appreciated that other sultaines may be used in combination with the lauryl hydroxysultaine. Illustrative further sultanes may be or include, but are not limited to, lauramidopropyl hydroxysultaine, cocamidopropyl hydroxysultaine, oleamidopropyl hydroxysultaine, tallowamidopropyl hydroxysultaine, or the like, or combinations thereof. In at least one implementation, the one or more sultaines excludes at least cocamidopropyl hydroxysultaine.

In at least one implementation, the at least one amphoteric or zwitterionic surfactant includes one or more betaine and excludes the sultaines. In a preferred implementation, the at least one amphoteric or zwitterionic surfactant includes one or more betaine and one or more sultaines. For example, the amphoteric or zwitterionic surfactant includes a combination of at least one betaine and at least one sultaine. In a more preferred implementation, the amphoteric or zwitterionic surfactant includes a combination of at least cocamidopropyl betaine and lauryl hydroxysultaine.

The surfactant system includes at least one anionic surfactant and at least one zwitterionic surfactant as defined in the claims. The at least one anionic surfactant includes sodium laureth sulfate (SLES) as defined in the claims, and the at least one amphoteric or zwitterionic surfactant includes cocamidopropyl betaine. The at least one anionic surfactant includes sodium laureth sulfate, and the at least one zwitterionic surfactant includes one or more of cocamidopropyl betaine and lauryl hydroxysultaine as defined in the claims. In an even more preferred implementation, the surfactant system includes a combination of, consists essentially of, or consists of sodium laureth sulfate, cocamidopropyl betaine, and lauryl hydroxysultaine.

The amount of each of the one or more surfactants present in the personal care composition or the surfactant system thereof may vary in accordance with the claims i.e. the surfactant system and the thickener are present in an amount of less than 10.0 wt% as further defined in the claims, based on the total weight of the personal care composition.

In a preferred implementation, the surfactant system may be present in an amount of from about 6.0 wt%, about 6.5 wt%, about 6.8 wt%, about 6.9 wt%, about 7.0 wt%, about 7.5 wt%, or about 8.0 wt% to 10 wt%, based on the total weight of the personal care composition. For example, the surfactant system may be present in an amount of from about 6.8 wt% to less than 10 wt%, about 7.0 wt% to less than 10 wt%, more preferably about 6.8 wt% to about 8.5 wt%, even more preferably about 7 wt% to about 7.5 wt%, based on the total weight of the personal care composition.

As discussed above, the surfactant system includes at least one anionic surfactant and at least one zwitterionic surfactant as defined in the claims, where the at least one anionic surfactant includes sodium laureth sulfate and the at least one zwitterionic surfactant includes one or more of cocamidopropyl betaine and lauryl hydroxysultaine. In at least one implementation, the sodium laureth sulfate is present in an amount as defined in the claims such as less than 7.5 wt%, or less than 6 wt%, based on the total weight of the personal care composition. The sodium laureth sulfate, is present in an amount of from 4 wt% to less than 7.8 wt%, about 4.5 wt% to about 6.5 wt%, more preferably about 5 wt% to about 5 wt%, or even more preferably about 5.6 wt%, based on the total weight of the personal care composition. The cocamidopropyl betaine, if present, is present in an amount of from 0.5 wt% to 2.0 wt%, preferably about 0.8 wt% to about 1.8 wt%, about 1 wt% to about 1.4 wt%, more preferably about 1.1 wt% to about 1.3 wt%, or more preferably about 1.2 wt%, based on the total weight of the personal care composition. Lauryl hydroxysultaine may be present in an amount of from 0.2 wt% to 1.0 wt%, preferably about 0.3 wt% to about 0.9 wt%, more preferably about 0.4 wt% to about 0.8 wt%, even more preferably about 0.5 wt% to about 0.7 wt%, or about 0.6 wt%, based on the total weight of the personal care composition.

For example, the total weight ratio of the anionic surfactants to the zwitterionic surfactants in the surfactant system may be from about 2:1 to about 4:1, about 2.5:1 to about 3.5:1, or about 3:1. In a preferred implementation, the weight ratio of the sodium laureth sulfate to the cocamidopropyl betaine and the lauryl hydroxysultaine may be from about 2:1 to about 4:1, about 2.5:1 to about 3.5:1, or about 3:1. In at least one implementation, the weight ratio of the sodium laureth sulfate to the cocamidopropyl betaine may be about 3.6:1 or greater, about 3.8:1 or greater, or about 4:1 or greater. In a preferred implementation, the weight ratio of the sodium laureth sulfate to the cocamidopropyl betaine may be from about 3.5:1 to about 6:1, about 4:1 to about 5.7:1, about 4.5:1 to about 5:1, or about 4.7:1. In another preferred implementation, the weight ratio of the sodium laureth sulfate to the lauryl hydroxysultaine may be from about 7:1 to about 11:1, about 8.5:1 to about 10:2, or more preferably about 9:1. In yet another preferred implementation, the weight ratio of the cocamidopropyl betaine to the lauryl hydroxysultaine may be from about 1.6:1 to about 3.5:1, about 2:1 to about 3:1, or about 2.2:1 to about 2.6:1, or more preferably about 2.4:1.

The personal care composition comprises less than 0.05 weight% of polyquaternium as defined in the claims, and is optionally free of cocomonoethanolamide (CMEA). Polyquaternium is the International Nomenclature for Cosmetic Ingredients designation for several polycationic polymers that are used in the personal care industry. Polyquaternium is a neologism used to emphasize the presence of quaternary ammonium centers in the polymer. INCI has approved at least 37 different polymers under the polyquaternium designation. Different polymers are distinguished by the numerical value that follows the word "polyquaternium". Polyquaternium-5, polyquaternium-7, and polyquaternium-47 are three examples, each a chemically different type of polymer. The numbers are assigned in the order in which they are registered rather than because of their chemical structure.

The one or more thickeners include esters of polyalkoxylated polyols and fatty acids as defined in the claims. The one or more thickeners may be or include a tetra ester made of fatty acids and a polyoxyethylene pentaerythritol having four hydrophilic poly-(ethylene glycol) arms. In a preferred implementation, the one or more thickeners include at least a polymer of ethylene glycol, such as PEG-150 pentaerythrityl tetrastearate, where all four hydrophilic poly-(ethelene glycol) arms may be capped with stearic fatty acids. Other suitable thickeners may be or include, but are not limited to, polyethylene glycol 6000 distearate, also known with INCI name of PEG-150 distearate; PEG 120 methyl glucose dioleate and PEG 120 methylglucose trioleate (GLU-COMATE^{™} DOE 120 and GLUCOMATE^{™} VLT); PEG-150 Pentaerythrityl Tetrastearate (CROTHIX^{™}, CROTHIX^{™} and VERSATHIX^{™}); PEG-150 Polyglyceryl-2 Tristerate (Genapol LT); PEG/PPG-120/10-Trimethlolpropane Trioleate (Arlypon TT). The number of hydrophilic polyalkoxylated arms is two for PEG-150 distearate, three for ARLYPON TT, four for Genapol LT and CROTHIX, CROTHIX Liquid, and VERSATHIX, and five for GLU-CORNATE DOE 120. In a preferred implementation, the thickener includes VER-SATHIX^{™} (INCI Name: PEG-150 Pentaerythrityl Tetrastearate (and) PPG-2 Hydroxyethyl Cocamide (and) Aqua), which is commercially available from Croda Inc., of Newark, New Jersey. VERSATHIX^{™} may have a pH of about 6.5 to about 7.5, a boiling point of about 100°C, and a density of about 1.054 g/ml at 25°C. VERSATHIX^{™} may include amides, coco, N-(hydroxyethyl), propoxylated (CAS Number: 201363-52-2), PEG-150 pentaerythrityl tetrastearate, and water.

The one or more thickeners are present in the personal care composition in an amount of 0.08 wt% to 0.12 wt% as defined in the claims. Generally, one or more thickeners may be present in the personal care composition in an amount of from greater than about 0.09 wt% to about 0.11 wt%, 0.12 wt%, based on the total weight of the personal care composition. In a preferred implementation, the one or more thickeners includes VERSATHIX^{™} (INCI Name: PEG-150 Pentaerythrityl Tetrastearate (and) PPG-2 Hydroxyethyl Cocamide (and) Aqua), which is commercially available from Croda Inc., of Newark, New Jersey, or one or more components thereof, and the VERSATHIX^{™} is present in an amount of from 0.08 wt% to 0.12 wt%, more preferably, about 0.09 wt% to about 0.11 wt%, even more preferably, about 0.10 wt%, based on the total weight of the personal care composition.

In a preferred implementation, the surfactant system and the one or more thickeners may be referred to as active ingredients or actives. The actives may be present in an amount of from about 6.0 wt%, about 6.5 wt%, about 6.8 wt%, about 6.9 wt%, about 7.0 wt%, about 7.2 wt%, or about 7.4 wt% to about 7.5 wt%, or about 8.0 wt%, about 8.5 wt%, about 9.0 wt%, about 9.5 wt%, or 10 wt%, based on the total weight of the personal care composition. For example, the actives may be present in an amount of from about 6.8 wt% to less than 10 wt%, about 7.0 wt% to less 10 wt%, more preferably about 6.8 wt% to about 8.5 wt%, even more preferably about 7 wt% to about 7.5 wt%, based on the total weight of the personal care composition. In at least one implementation, the actives may be present in an amount of greater than 5 wt%, greater than 6 wt%, greater than 6.5 wt%, greater than 7 wt%, or greater than 7.2 wt% and less than 10 wt%, based on the total weight of the personal care composition.

For example, the weight ratio of the anionic surfactant, which is sodium laureth sulfate, to the thickener may be from about 45:1 to about 70:1, about 50:1 to about 65:1, preferably about 55:1 to about 60:1, more preferably about 56:1 to about 57:1. The weight ratio of the anionic surfactant to the thickener may also be about 44:1 or greater, or about 46:1 or greater. In another example, the weight ratio of the betaine (i.e. cocamidopropyl betaine) to the thickener may be from about 9.5:1 to about 16:1, more preferably about 10:1 to about 15:1, even more preferably about 12:1 to about 14:1, even more preferably about 12:1. In at least one implementation, the ratio of the anionic surfactant to the thickener may be at least 44:1 or greater or 45:1 or greater, and the ratio of the anionic surfactant to the zwitterionic surfactant may be from about 7.2:1 to about 10:1. In yet another implementation, the ratio of the anionic surfactant to the thickener may be at least 55:1 or greater or 56:1 or greater, and the ratio of the anionic surfactant to the zwitterionic surfactant may be from about 9:1 to about 11:1.

In at least one implementation, the personal care composition may include one or more salts or a solution including the one or more salts (e.g., brine solution). Any suitable salts may be utilized, including, but not limited to, sodium salts (e.g., sodium chloride), potassium salts (potassium chloride), or the like, or combinations thereof. The salt solutions may be provided as 25% salt solutions. The one or more salts or a solution thereof may be capable of or configured to modify (e.g., increase or decrease) the viscosity of the personal care composition. It should be appreciated that the one or more salts or a solution thereof are generally utilized to modify the anionic surfactants of the personal care composition to thereby modify the viscosity of the personal care composition. In at least one implementation, the personal care compositions disclosed herein may include the anionic surfactants in an amount less than 6.5 wt%, less than 6.2 wt%, less than 6 wt%, less than 5.8 wt%, or less than or equal to about 5.6 wt%, based on the total weight of the personal care composition. In another example, the personal care composition includes a maximum amount of the anionic surfactants of about 6.5 wt%, about 6.2 wt%, about 6 wt%, about 5.8 wt%, or about 5.6 wt%, based on the total weight of the personal care composition. In view of the limited amount of the anionic surfactant, it should be appreciated that the maximum effective amount of the one or more salts or the solution thereof to modify the viscosity may also be limited and at least partially determined by the amount of the anionic surfactants present in the personal care composition. In at least one implementation, the salt is provided as a 25% solution, and the 25% solution is provided in an amount of from greater than 0 wt% to less than about 5 wt%, less than about 4.5 wt%, less than about 4 wt%, less than about 3.75 wt%, less than about 3.5 wt%, less than about 3 wt%, less than about 2.5 wt%, or less than about 2 wt%, based on the total weight of the personal care composition. It should be appreciated that the salt solution in an amount of about 5 wt% (of a 25% salt solution) may provide a viscosity of about 8,000 mPa·s (cP) or greater.

In at least one implementation, the personal care composition may include one or more humectants. Illustrative humectants may be or include, but are not limited to, glycerin (e.g., vegetable refined glycerin, etc.), propylene glycol, polyethylene glycol, ascorbic acid, ascorbyl dipalmitate, acetamide MEA, caprylyl glycol, glucose glutamate, glucuronic acid, TEA-lactate, TEA-PCA, corn syrup, fructose, glucose, glycerin, glycol, 1,2,6-hexanetriol, sodium lactate, sodium PCA, hydrogenated starch hydrolysate, inositol, lactic acid, lactose, mannitol, PCA, PEG-10 propylene glycol, polyamino sugar condensate, pyridoxine dilaurate, saccharide hydrolysate, hydroxystearyl methylglucamine, glucamine, maltitol, mannitol, methyl gluceth-10, methyl gluceth-20, riboflavin, PEG-4, PEG-6, PEG-8, PEG-9, PEG-10, PEG-12, PEG-14, PEG-16, PEG-18, PEG-20, PEG-32, PEG-40, glutamic acid, glycereth-7, glycereth-12, glycereth-26, saccharide isomerate, sorbeth-20, sorbitol, sucrose, thioglycerin, tris-(hydroxymethyl)nitromethane, tromethamine, histidine, polymers of ethylene glycol (PEG), PEG-75, PEG-135, PEG-150, PEG-200, PEG-5 pentaerythritol ether, polyglyceryl sorbitol, sorbitol, urea, xylitol, or the like, or combinations thereof. In a preferred implementation, the humectants may be or include, but is not limited to, glycerin, caprylyl glycol, or combinations thereof.

The one or more humectants may be present in an amount of from about 0.1 weight % to about 10 weight % or from about 0.5 weight % to about 20 weight %, based on a total weight of the personal care composition. For example, any one or more of the humectants may be present in an amount of from about 0.1 weight %, about 0.2 weight %, about 0.3 weight %, about 0.4 weight %, about 0.5 weight %, about 1 weight %, about 1.5 weight %, about 2 weight %, about 2.5 weight %, about 3 weight %, about 3.5 weight %, about 4 weight %, about 4.5 weight %, or about 5 weight % to about 5.5 weight %, about 6 weight %, about 6.5 weight %, about 7 weight %, about 7.5 weight %, about 8 weight %, about 8.5 weight %, about 9 weight %, about 9.5 weight %, about 10 weight %, or about 20 weight %, based on a total weight of the personal care composition. In a preferred implementation, the humectant includes glycerin in an amount of from about 0.8 to about 1.5 weight %, about 1 weight % to about 1.4 weight %, about 1.1 weight % to about 1.3 weight %, or about 1.25 weight %, based on the total weight of the personal care composition.

In at least one implementation, the personal care composition may include one or more preservatives in an amount of greater than 0 weight % and less than or equal to about 3 weight %, less than or equal to about 2.5 weight %, less than or equal to about 2 weight %, less than or equal to about 1.5 weight %, less than or equal to about 1 weight %, less than or equal to about 0.75 weight %, less than or equal to about 0.5 weight %, or less than or equal to about 0.25 weight %, based on a total weight of the personal care composition. Illustrative preservatives may include, but are not limited to, benzalkonium chloride; benzethonium chloride, 5-bromo-5-nitro-1,3-dioxane; 2-bromo-2-nitropropane-1,3-diol; alkyl trimethyl ammonium bromide; N-(hydroxymethyl)-N-(1,3-dihydroxy methyl-2,5-dioxo-4-imidaxolidinyl-N-(hydroxy methyl)urea; 1-3-dimethyol-5,5-dimethyl hydantoin; formaldehyde; iodopropynl butyl carbamate, butyl paraben; ethyl paraben; methyl paraben; propyl paraben, mixture of methyl isothiazolinone/methyl-chloroisothiazoline in a 1:3 wt. ratio; mixture of polyvinylpyrro/butyl paraben/methyl paraben/propylparaben; 2-phenoxyethanol; tris-hydroxyethyl-hexahydrotriaz-ine; methylisothiazolinone; 5-chloro-2-methyl-4-isothiazolin-3-one; 1,2-dibromo-2,4-dicyanobutane; 1-(3-chloroalkyl)-3,5,7-triaza-azoniaadam-antane chloride; sodium benzoate; organic acids, sorbic acid, lactic acid, citric acid, or the like, or any combination thereof. In a preferred implementation, the one or more preservatives include sodium benzoate in an amount of from greater than 0 weight % to less than or equal to 2 weight %, greater than 0 weight % to less than or equal to 1 weight %, or about 0.25 weight %, based on the total weight of the personal care composition.

In at least one implementation, the personal care composition may include one or more acids, one or more bases, and/or one or more buffers configured to adjust or control the pH of the personal care composition. The one or more acids, one or more bases, and/or one or more buffers may, separately and independently, be present in an amount of from greater than 0 weight % to less than or equal to about 5 weight %, less than or equal to about 4 weight %, less than or equal to about 3 weight %, less than or equal to about 2 weight %, less than or equal to about 1 weight %, less than or equal to about 0.75 weight %, less than or equal to about 0.5 weight %, less than or equal to about 0.4 weight %, or less than or equal to about 0.35 weight %, based on the total weight of the personal care composition. Illustrative bases may include, but are not limited to, ammonia; mono-, di-, and tri-alkyl amines; mono-, di-, and tri-alkanolamines; alkali metal and alkaline earth metal hydroxides; sodium hydroxide, potassium hydroxide, lithium hydroxide, monoethanolamine, triethylamine, isopropanolamine, diethanolamine, triethanolamine, or the like, or combinations thereof. Illustrative acids may include, but are not limited to, mineral acids, such as hydrochloric acid, nitric acid, phosphoic acid, and sulfuric acid, organic acids, polycarboxylic acids, such as citric acid, glycolic acid, and lactic acid, or the like, or combinations thereof. In a preferred implementation, the personal care composition includes at least one acid, such as citric acid, and the citric is present in an amount of from greater than 0 weight % to about 0.8 weight %, from about 0.4 weight % to about 0.6 weight %, or about 0.5 weight %, based on the total weight of the personal care composition.

The personal care composition may include water. The water may be deionized water, demineralized water, and/or softened water. Water may make up the balance of the personal care composition. For example, the amount of water present in the personal care composition may be greater than 60 weight %, greater than 65 weight %, greater than 70 weight %, greater than 75 weight %, greater than 80 weight %, greater than 85 weight %, greater than 90 weight %, greater than 92 weight %, greater than 94 weight %, based on the total weight of the personal care composition. The amount of water in the personal care composition may include free water added and water introduced with other components or materials of the personal care composition. For example, the amount of the water in the personal care composition may include free water and water associated with one or more surfactants and/or any other components of the personal care composition.

The personal care compositions may include one or more emollients. The one or more emollients may be capable of or configured to maintain a soft, smooth, and pliable appearance to the skin. The one or more emollients may be or include, but are not limited to, fatty esters, fatty alcohols, or combinations thereof. Illustrative emollients may be or include, but are not limited to, diisopropyl adipate, oleyl alcohol, lanolin, isopropyl myristate, isopropyl palmitate, coco-caprylate, diethylhexyl carbonate, caprylic/capric triglycerides, cetyl lactate, cetyl palmitate, hydrogenated castor oil, glyceryl esters, hydroxycetyl isostearate, hydroxy cetyl phosphate, isopropyl isostearate, isostearyl isostearate, diisopropyl sebacate, polyoxypropylene (5) poloxyethylene (20) cetyl ether (PPG-5-Ceteth-20), 2-ethylhexyl isononoate, 2-ethylhexyl stearate, C₁₂ to C₁₆ fatty alcohol, C₁₂ to C₁₆ fatty alcohol lactate, isopropyl lanolate, 2-ethyl-hexyl salicylate, or the like, or combinations thereof. In some implementations, the one or more emollients may be a combination of fatty alcohols. In some implementations, the one or more emollients may be 1-hexadecanol, acetylated lanolin, behenocyl dimethicone, C₁₂-₁₅ alkyl benzoate, cetearyl octanoate, cocoglycerides, dicaprylate/dicaprate dimethicone copolyol, dimethiconol, dioctyl adipate, glyceryl stearate, isocetyl alcohol, isohexadecane, isopentylcyclohexanone, isopropyl palmitate, lauryllactate, mineral oil, methoxy peg-22/dodecyl glycol copolymer, myristyl lactate, ocryldodecyl neopentanoate, octyl cocoate, octyl palmitate, octyl stearate, octyldodecyl neopentanoate, polyglyceryl-4 isosterate, polyoxyl 40 stearate, polyoxymethylene urea, potassium sorbate, propylene glycol, propylene glycol isoceth-3 acetate, propylene glycol myristyl ether acetate, or combinations thereof. Illustrative emollients may also include, but are not limited to, a high molecular weight saturated and unsaturated fatty alcohol such as, but not limited to, carbitol, lauryl alcohol, myristyl alcohol, cetyl alcohol, isocetyl alcohol, stearyl alcohol, isostearyl alcohol, hydroxystearyl alcohol, oleyl alcohol, ricinoleyl alcohol, behenyl alcohol, erucyl alcohol, 2-octyldodecanyl alcohol, cetearyl alcohol, lanolin alcohol, or the like, or any combination thereof.

In at least one implementation, the personal care composition may include one or more emollients in an amount from greater than 0 weight % to about 5 weight %, based on the total weight of the personal care composition. For example, the one or more emollients may be present in an amount of from greater than 0 weight %, about 0.1 weight %, about 0.3 weight %, about 0.5 weight %, about 1 weight %, or about 2 weight % to about 2.5 weight %, about 3 weight %, about 3.5 weight %, about 4 weight %, or about 5 weight %, based on the total weight of the personal care composition.

In some implementations, the personal care composition may include one or more skin care agents. Any suitable skin care agents that do not adversely affect the stability and/or efficacy of the personal care composition may be utilized. The skin care agents may generally include one or more polymers (e.g., polyvinylpyrrolidone), starches (e.g., tapioca starches, hydrophobically modified corn starch, such as DRY-FLO TS^{®} CAS Nos. 68989-12-8, 68554-70-1, 9005-25-8, etc.), protein derivatives (e.g., derivatized hydrolyzed wheat protein), ethoxylated fatty ethers, cellulosics (e.g., hydroxyethylcellulose), or the like, or combinations thereof. Illustrative skin care agents may include, but are not limited to, esters comprising an aliphatic alcohol having about 2 to about 18 carbon atoms condensed with an aliphatic or aromatic carboxylic acid including about 8 to about 20 carbon atoms (e.g., isopropyl myristate, decyl oleate, cetearyl isononanate, etc.). The esters may be straight chained or branched. In a preferred implementation, the ester has a molecular weight of less than about 500.

Other skin care agents may include, but are not limited to, polyvinylpyrrolidone, polyquaternium-4, polyquaternium-7, polyquaternium-10, guar gum derivatives, hydroxypropylmethylcellulose, hydroxyethylcellulose, a polyethylene glycol, a methyl ether of a polyethylene glycol, quaternium-79, wheat germamidopropyl hydroxypropyl dimonium hydrolyzed wheat protein, stearyl methicone, dimethicone copolyol, dimethicone propyl PG betaine, poly(sodium styrene sulfonate), sorbitan oleate, steareth-2, steareth-21, isoceteth-20, PEG-7 glyceryl cocoate, PEG-75 lanolin, glycereth-26, PPG-5-ceteth-20, a C₁₂-C₂₀ alcohol, canola oil, glyceryl laurate, triglyceryl monostearate, glyceryl monostearate, vitamin E acetate, sunflower seed amidopropylethyldimonium ethylsulfate, sodium PEG-7 olive oil carboxylate, PPG-1 hydroxyethyl caprylamide, PPG-2 hydroxyethyl cocamide, mineral oil, petrolatum, aloe barbadensis, isostear-amidopropylmorpholine lactate, strontium acetate, palmitamidopropyltrimonium chloride, or the like, or combinations thereof.

The personal care composition may include one or more additional ingredients. Illustrative additional ingredients may include, but are not limited to, one or more dyes, fragrances, preservatives, antioxidants, chelating agents (e.g., EDTA, phosphates, etc.), opacifiers, hydric solvents, hydrotropes, antimicrobials, or the like, or any combination thereof.

All ingredients for use in the compositions described herein are topically acceptable. As used herein, "topically acceptable" may refer any ingredient that is present in a composition as described in an amount and form which does not render the composition unsafe for use on surfaces of skin.

### METHODS

The present disclosure may provide methods for preparing stable personal care compositions as defined in the claims. The personal care compositions may have or exhibit comparable, enhanced, or relatively greater stability as compared to conventional personal care compositions that include a surfactant system in an amount of from about 10 wt% or greater, based on the total weight of the conventional personal care composition. For example, the personal care compositions disclosed herein may exhibit no phase separation, relatively stable pH, and/or relatively stable viscosity when exposed to various accelerated aging conditions as compared to conventional personal care compositions. It should be appreciated by one having ordinary skill in the art that the aging conditions may be provided or defined by the International Council for Harmonisation of Technical Requirements for Pharmaceuticals for Human Use (ICH) Guidelines. The method may include mixing, combining, stirring, emulsifying, or otherwise contacting two or more of the components/ingredients disclosed herein with one another. The method may also include heating the components/ingredients before, during, or after mixing, combining, stirring, emulsifying, or otherwise contacting the components with one another.

The present disclosure may provide methods for preparing personal care compositions having or exhibiting comparable, enhanced, or relatively greater sensory properties as compared to conventional personal care compositions including a surfactant system present in an amount of from about 10 wt% or greater, based on the total weight of the conventional personal care composition. The method may include mixing, combining, stirring, emulsifying, or otherwise contacting two or more of the components/ingredients disclosed herein with one another. The method may also include heating the components/ingredients before, during, or after mixing, combining, stirring, emulsifying, or otherwise contacting the components with one another.

### EXAMPLES

The examples and other implementations described herein are exemplary and not intended to be limiting in describing the full scope of compositions and methods of this disclosure as defined by the appended claims.

### Example 1

Liquid personal care compositions (1)-(27) were prepared by combining the ingredients/components according to Tables 1A and 1B, where the values are wt% based on the total weight of the respective composition. Particularly, one or more of water, sodium laureth sulfate (70% solution), cocamidopropyl betaine (30% solution), lauryl hydroxysultaine (37.5% solution), VERSATHIX^{™} (commercially available from Croda Inc. of Edison, New Jersey, and including PEG-150 Pentaerythrityl Tetrastearate, PPG-2 Hydroxyethyl Cocamide, and water), brine (25% salt solution), a polyquat, an opacifier, minors, or combinations thereof were combined according to Table 1A and 1B. The ratio of active ingredients in each of the liquid personal care compositions (1)-(27) is summarized in Tables 2A and 2B.

**Table 1A**

| **Composition of Liquid Personal Care Compositions (1)-(14)** | | | | | | | | | | | | | | |
|---|---|---|---|---|---|---|---|---|---|---|---|---|---|---|
| | 1* | 2 | 3 | 4 | 5 | 6 | 7 | 8 | 9 | 10 | 11 | 12 | 13 | 14 |
| Water | 84.6 | 80.7 | 79.3 | 82.0 | 79.6 | 80.9 | 80.1 | 80.4 | 80.1 | 81.2 | 79.6 | 80.9 | 81.7 | 82.0 |
| SLES¹ | 11.1 | 8.00 | 8.8 | 7.2 | 8.8 | 7.2 | 8.8 | 8.8 | 8.8 | 7.2 | 8.8 | 7.2 | 7.2 | 7.2 |
| CAPB² | 7.7 | 4.00 | 4.4 | 3.6 | 4.4 | 4.4 | 3.6 | 3.6 | 3.6 | 4.4 | 4.4 | 4.4 | 3.6 | 3.6 |
| Sultaine³ | -- | 1.68 | 1.85 | 1.51 | 1.51 | 1.85 | 1.85 | 1.51 | 1.85 | 1.51 | 1.51 | 1.85 | 1.85 | 1.51 |
| Thickener⁴ | -- | 0.10 | 0.09 | 0.11 | 0.11 | 0.11 | 0.09 | 0.11 | 0.11 | 0.11 | 0.09 | 0.09 | 0.09 | 0.09 |
| CMEA⁵ | 0.5 | -- | -- | -- | -- | -- | -- | -- | -- | -- | -- | -- | -- | -- |
| Brine | 2.4 | 2.0 | 2.75 | 4.3 | 2.5 | 2.3 | 3.35 | 3.5 | 3 | 3 | 2.7 | 2.8 | 3.75 | 4.9 |
| Polyquat | -- | -- | -- | -- | -- | -- | -- | -- | -- | -- | -- | -- | -- | -- |
| Opacifier | -- | -- | -- | -- | -- | -- | -- | -- | -- | -- | -- | -- | -- | -- |
| Minors | Q.C. | Q.C. | Q.C. | Q.C. | Q.C. | Q.C. | Q.C. | Q.C. | Q.C. | Q.C. | Q.C. | Q.C. | Q.C. | Q.C. |
| Total Actives | 10.0 8 | 7.53 | 8.26 | 6.80 | 8.16 | 7.16 | 8.02 | 7.92 | 8.04 | 7.04 | 8.14 | 7.14 | 6.90 | 6.78 |

| | | | | | | | | | | | | | | |
|---|---|---|---|---|---|---|---|---|---|---|---|---|---|---|
| ¹ Sodium laureth Sulfate (70% solution) ² Cocamidopropyl Betaine (30% solution) ³ Lauryl Hydroxysultaine (37.5% solution) ⁴ VERSATHIX^{™} commercially available from Croda Inc. of Edison, New Jersey, including PEG-150 Pentaerythrityl Tetrastearate, PPG-2 Hydroxyethyl Cocamide, and water ⁵ Cocomonoethanolamide ⁶ 25% Salt solution *: comparative | | | | | | | | | | | | | | |

**Table 1B**

| **Composition of Comparative Personal Care Compositions (15)-(27)** | | | | | | | | | | | | | |
|---|---|---|---|---|---|---|---|---|---|---|---|---|---|
| | 15* | 16* | 17* | 18* | 19* | 20 | 21 | 22* | 23* | 24 | 25 | 26 | 27 |
| Water | 82.0 | 85.4 | 84.2 | 89.2 | 89.2 | 89.6 | 87.8 | 89.4 | 90.6 | 93.7 | 93.3 | 89.8 | 92.8 |
| SLES¹ | 8 | 4.5 | 4.5 | 8 | 8 | 5.6 | 6.4 | 7.2 | 7.2 | 7.2 | 7.2 | 4 | 4 |
| CAPB² | 4 | 4 | 4 | 4 | 4 | 2.8 | 3.2 | 3.6 | 3.6 | 3.6 | 3.6 | 4.5 | 4.5 |
| Sultaine³ | -- | 1 | 1 | -- | -- | 0.7 | 1.2 | 1.35 | 1.35 | 1.35 | 1.35 | 1 | 1 |
| Thickener⁴ | 0.2 | 0.2 | 0.2 | 0.15 | 0.15 | 0.1 | 0.12 | 0.14 | 0.14 | 0.09 | 0.09 | 0.09 | 0.09 |
| CMEA⁵ | -- | -- | -- | -- | -- | -- | -- | -- | -- | -- | -- | -- | -- |
| Brine⁶ | 4 | 4 | 3.5 | 4 | 5 | 0-4 | 0-4 | 4.3 | 2.7 | -- | -- | 2 | -- |
| Polyquat | -- | -- | 1.25 | 1.25 | -- | -- | -- | -- | -- | -- | -- | 1.25 | -- |
| Opacifier | -- | -- | 1 | 1 | -- | -- | -- | -- | -- | -- | -- | 1 | -- |
| Minors | Q.C. | Q.C. | Q.C. | Q.C. | Q.C. | Q.C. | Q.C. | Q.C. | Q.C. | Q.C. | Q.C. | Q.C. | Q.C. |
| Total Actives | 6.90 | 4.35 | 4.82 | 6.91 | 6.91 | 5.13 | 5.98 | 6.74 | 6.74 | 6.74 | 6.72 | 4.62 | 4.62 |

| | | | | | | | | | | | | | |
|---|---|---|---|---|---|---|---|---|---|---|---|---|---|
| ¹ Sodium laureth Sulfate (70% solution) ² Cocamidopropyl Betaine (30% solution) ³ Lauryl Hydroxysultaine (37.5% solution) ⁴ VERSATHIX^{™} commercially available from Croda Inc. of Edison, New Jersey, including PEG-150 Pentaerythrityl Tetrastearate, PPG-2 Hydroxyethyl Cocamide, and water ⁵ Cocomonoethanolamide ⁶ 25% Salt solution *: comparative | | | | | | | | | | | | | |

**Table 2A**

| **Ratio of Active Ingredients in Compositions (1)-(14)** | | | | | | | | | | | | | | |
|---|---|---|---|---|---|---|---|---|---|---|---|---|---|---|
| Ratios | 1 | 2 | 3 | 4 | 5 | 6 | 7 | 8 | 9 | 10 | 11 | 12 | 13 | 14 |
| SLES:CAPB | 3.4 | 4.7 | 4.7 | 4.7 | 4.7 | 3.8 | 5.7 | 5.7 | 5.7 | 3.8 | 4.7 | 3.8 | 4.7 | 4.7 |
| SLES:Sultaine | -- | 8.9 | 8.9 | 8.9 | 10.9 | 7.3 | 8.9 | 10.9 | 8.9 | 8.9 | 10.9 | 7.3 | 7.3 | 8.9 |
| SLES:thickener | -- | 56.0 | 68.4 | 45.8 | 56.0 | 45.8 | 68.4 | 56.0 | 56.0 | 45.8 | 68.4 | 56.0 | 56.0 | 56.0 |
| CAPB:Sultaine | -- | 2.4 | 2.4 | 2.4 | 2.9 | 2.4 | 1.9 | 2.4 | 1.9 | 2.9 | 2.9 | 2.4 | 1.9 | 2.4 |
| CAPB:thickener | -- | 12.0 | 14.7 | 9.8 | 12.0 | 12.0 | 12.0 | 9.8 | 9.8 | 12.0 | 14.7 | 14.7 | 12.0 | 12.0 |
| Sultaine:thickener | -- | 6.30 | 7.73 | 5.14 | 5.14 | 6.30 | 7.73 | 5.14 | 6.30 | 5.14 | 6.30 | 7.73 | 7.73 | 6.30 |

**Table 2B**

| **Ratio of Active Ingredients in Compositions (15)-(27)** | | | | | | | | | | | | | |
|---|---|---|---|---|---|---|---|---|---|---|---|---|---|
| Ratios | 15 | 16 | 17 | 18 | 19 | 20 | 21 | 22 | 23 | 24 | 25 | 26 | 27 |
| SLES:CAPB | 4.7 | 2.6 | 2.6 | 4.7 | 4.7 | 4.7 | 4.7 | 4.7 | 4.7 | 4.7 | 4.7 | 2.1 | 2.1 |
| SLES:Sultaine | -- | -- | 8.4 | -- | -- | 14.9 | 10.0 | 10. 0 | 10. 0 | 10.0 | 10.0 | 7.5 | 7.5 |
| SLES: thickener | 56.0 | -- | 35.0 | 50. 9 | 50. 9 | 35.6 | 49.8 | 45. 8 | 45. 8 | 45.8 | 56.0 | 31.1 | 31.1 |
| CAPB:Sultaine | -- | -- | 4.0 | -- | -- | 4.0 | 2.7 | 2.7 | 2.7 | 2.7 | 2.7 | 4.5 | 4.5 |
| CAPB:thickener | 12.0 | -- | 13.3 | 10. 9 | 10. 9 | 7.6 | 10.7 | 9.8 | 9.8 | 9.8 | 12.0 | 15.0 | 15.0 |
| Sultaine:thickener | -- | -- | 1.88 | -- | -- | 2.63 | 3.75 | 3.6 | 3.6 | 5.63 | 5.63 | 4.16 | 4.16 |

### Example 2

The stability of each of the personal care compositions (1)-(27) prepared in Example 1 was evaluated. Particularly, the stability of each of the personal care compositions (1)-(27) of Example 1 was evaluated by measuring or observing the pH and viscosity under various accelerated aging conditions.

To evaluate the viscosity under the various accelerated aging conditions, each of the personal care compositions (1)-(27) was placed in a respective glass jar and exposed to an environment maintained at the particular conditions (i.e., time, relative humidity [RH], and temperature) indicated in Table 3. After the respective times of each of the studies elapsed, each of the personal care compositions (1)-(27) was removed from the environment and allowed to cool in a monitored stability chamber maintained at about 25°C. After cooling to about 25°C, the viscosity and pH were measured, and the personal care compositions (1)-(27) were observed/evaluated visually. Viscosity was measured using a Brookfield rheometer with spindle T-93 at 10 RPM for about 1 minute.

The results of the pH and viscosity stability analysis of the personal care compositions (1)-(27) are summarized in Table 3. It should be appreciated that the personal care composition was considered relatively stable when the viscosity was maintained at about 4,000 mPa·s (cP) or greater or about 5,000 mPa·s (cP) or greater and/or the pH was maintained at about 5.1.

**Table 3**

| **Aging/Stability of Compositions (1)-(27)** | | | | |
|---|---|---|---|---|
| | 4 weeks | 4 weeks | 13 weeks | 13 weeks |
| | 40°C / 75%RH (mPa·s (cP)) / (pH) | 49°C (mPa·s (cP)) / (pH) | 25°C / 60%RH (mPa·s (cP)) / (pH) | 40°C / 75%RH (mPa·s (cP)) / (pH) |
| 1 | 5700 / pH 5.1 | 6400 / pH 5.0 | 5500 / pH 5.0 | 5980 / pH 5.1 |
| 2 | 6800 / pH 5.07 | 7560/ pH 5.13 | 6540/ pH 5.07 | 7880/ pH 5.09 |
| 3 | 6400 / pH 5.20 | 7140 / pH 5.30 | 6560 /pH 5.24 | 7320 / pH 5.15 |
| 4 | 6100 / pH 5.19 | 6180 / pH 5.05 | 6000 /pH 5.05 | 6380 / pH 5.00 |
| 5 | 6260 / pH 5.20 | 7340 / pH 5.21 | 5680 /pH 5.12 | 6600 / pH 5.11 |
| 6 | 6340 / pH 5.09 | 7380 / pH 5.19 | 5940 /pH 5.09 | 6820 / pH 5.04 |
| 7 | 6220 / pH 5.09 | 7320 / pH 5.08 | 6480/ pH 5.06 | 7280 / pH 5.11 |
| 8 | 5800 / pH 5.11 | 6880 / pH 5.15 | 6000/ pH 5.11 | 6960 / pH 5.04 |
| 9 | 5760 / pH 5.11 | 7220 / pH 5.17 | 6540 / pH 5.13 | 7180 / pH 5.11 |
| 10 | 6400 / pH 5.01 | 6840 / pH 5.06 | 6040 / pH5.01 | 7440 / pH 5.03 |
| 11 | 6020 / pH 5.09 | 7120 / pH 5.13 | 6760 /pH 5.08 | 7220 / pH 5.07 |
| 12 | 6280 / pH 5.06 | 7340 / pH 5.13 | 6080 / pH 5.03 | 7440 / pH 5.07 |
| 13 | 5740 / pH 5.07 | 6580 / pH 5.07 | 6720 /pH 5.02 | 6840 / pH 5.00 |
| 14 | 3600 / pH 5.07 | 4320 / pH 5.17 | 3760 / pH 5.09 | 4780 / pH 5.06 |
| 15 | 1180 / pH 5.00 | 1220 / 5.02 | N/A¹ | N/A¹ |
| 16 | 5280 / pH 5.05 | 3860 / 5.02 | N/A¹ | N/A¹ |
| 17 | 4900 / pH 5.03 | 4160 / 4.94 | N/A¹ | N/A¹ |
| 18 | 840 / pH 5.27 | 1080 / 5.25 | 820 / pH 4.95 | 640 / pH 5.40 |
| 19 | 1140 / pH 5.14 | 880 / 5.16 | 1080 / pH 5.33 | 560 / pH 5.32 |
| 20 | N/A¹ | N/A¹ | N/A¹ | N/A¹ |
| 21 | N/A¹ | N/A¹ | N/A¹ | N/A¹ |
| 22 | 5600 / pH 5.02 | 5000 / pH 5.03 | N/A¹ | N/A¹ |
| 23 | 5260 / pH 4.92 | 4580 / pH 4.93 | N/A¹ | N/A¹ |
| 24 | 7760 / pH 5.01 | 6560 / pH 5.23 | 5500 / pH 4.98 | 5220 / pH 4.94 |
| 25 | 4880 / pH 5.12 | 5100 / pH 5.19 | 4860 / pH 4.96 | 4720 / pH 5.01 |
| 26 | N/A¹ | N/A¹ | N/A¹ | N/A¹ |
| 27 | N/A¹ | N/A¹ | N/A¹ | N/A¹ |

| | | | | |
|---|---|---|---|---|
| ¹Composition was not suitable for Aging/Stability study; | | | | |

As indicated in Table 3, the personal care compositions (1)-(13), (24), and (25) generally provided relatively stable backbones or personal care compositions as they maintained a viscosity of about 4,000 mPa·s (cP) or greater or about 5,000 mPa·s (cP) or greater and a pH of about 5.1 under various accelerated aging conditions. The personal care composition (14) had viscosity relatively close to the desired or target viscosity of about 4,000 mPa·s (cP), but was lower than the target viscosity. As further indicated in Table 3, personal care compositions (15)-(17), (20)-(23), (26), and (27) were not stable enough to be evaluated under each of the accelerated aging conditions; and thus, did not pass the stability study. Further, the personal care compositions (18) and (19) did not exhibit sufficient viscosity with respect to stability.

### Example 3

The stability of each of the personal care compositions (1)-(27) prepared in Example 1 was evaluated by measuring or observing the pH and viscosity under freeze-thaw conditions. The freeze temperature was either about -10°C or about -30°C and the thaw temperature was about 25°C at about 60% relative humidity. The cycle of freezing and thawing (i.e., about 24 hours of freezing and about 24 hours of thawing) was repeated three times for each of the compositions tested. To conduct a fast cycle, the sample was placed in the freezer for 24 hours then 25°C for 24 hours. The slow cycle was conducted by placing the sample in about -30°C for two (2) days, then moved to about -10°C for two (2) days, then moved to about 4°C for two (2) days, then moved to about 25°C for two (2) days. The viscosity and pH was measured after subjecting the composition to three cycles of freezing and thawing.

The results of the pH and viscosity stability analysis of the personal care compositions (1)-(27) are summarized in Table 4. It should be appreciated that the personal care composition was considered relatively stable when the viscosity was maintained at about 4,000 mPa·s (cP) or greater or about 5,000 mPa·s (cP) or greater and/or the pH was maintained at about 5.1.

**Table 4**

| **Aging/Stability of Compositions (1)-(27)** | | | |
|---|---|---|---|
| | Freeze/Thaw | Freeze/Thaw | Freeze/Thaw |
| | -10°C Fast (mPa·s (cP)) / (pH) | -30°C Fast (mPa·s (cP)) / (pH) | -30°C Slow (mPa·s (cP)) / (pH) |
| 1 | 5000 / pH 5.0 | 5080 / pH 5.1 | 5,800 pH 5.0 |
| 2 | 8280/ pH 5.13 | 5860/ pH 5.15 | 5980/ pH 5.15 |
| 3 | 6360 / pH 5.22 | 8300 / pH 5.28 | 6380 / pH 5.24 |
| 4 | 6660 / pH 5.13 | 6840 / pH 5.08 | 6640 / pH 5.12 |
| 5 | 8120 / pH 5.29 | 7160 / pH 5.19 | 7260 / pH 5.19 |
| 6 | 7660 / pH 5.24 | 8180 / pH 5.14 | 7020 / pH 5.15 |
| 7 | 8100 / pH 5.19 | 6580 / pH 5.13 | 6880 / pH 5.15 |
| 8 | 8120 / pH 5.24 | 5980 / pH 5.10 | 6750 / pH 5.17 |
| 9 | 8300 / pH 5.23 | 6680 / pH 5.08 | 6500 / pH 5.13 |
| 10 | 7340 / pH 5.20 | 8000 / pH 5.08 | 7200 / pH 5.17 |
| 11 | 6520 / pH 5.21 | 6640 / pH 5.18 | 6200 / pH 5.19 |
| 12 | 6820 / pH 5.18 | 5860 / pH 5.17 | 5880 / pH 5.17 |
| 13 | 6480 / pH 5.17 | 6080 / pH 5.15 | 6000 / pH 5.15 |
| 14 | 5580 / pH 5.22 | 4540 / pH 5.14 | 5680 / pH 5.15 |
| 15 | 1480 / pH 4.99 | 1600 / pH 5.09 | 1680 / pH 5.09 |
| 16 | 4120 / pH 4.96 | 880 / pH¹ | 4600 / pH¹ |
| 17 | 4380 / pH¹ | 1280 / pH¹ | 2880 / pH¹ |
| 18 | 860 / pH 5.29 | 680 / 5.41 | 800 / pH 5.44 |
| 19 | 1640 / pH 5.29 | 880 / 5.34 | 1020 / pH 5.35 |
| 20 | N/A² | N/A² | N/A² |
| 21 | N/A² | N/A² | N/A² |
| 22 | 1640 / pH 5.04 | 420 / pH 5.03 | 780 / pH 5.04 |
| 23 | 3780 / pH 5.22 | 2540 / pH 5.14 | 2940 / pH 5.22 |
| 24 | 4780 / pH 4.87 | 4680 / pH 5.10 | 1800 / pH 4.96 |
| 25 | 4340 / pH 5.00 | 2180 / pH 5.16 | 4860 / pH 5.12 |
| 26 | 1680 / pH 5.25 | 1140 / pH 5.30 | 1260 / pH 5.32 |
| 27 | 1420 / pH 4.90 | 720 / pH 4.95 | 1980 / pH 4.94 |

| | | | |
|---|---|---|---|
| ¹pH was not measured ²Composition was not suitable for Aging/Stability study | | | |

As indicated in Table 4, the personal care compositions (1)-(14) generally provided relatively stable backbones or personal care compositions as they maintained a viscosity of about 4,000 mPa·s (cP) or greater and/or a pH of about 5.1 under various accelerated aging conditions. As further indicated in Table 4, personal care compositions (15)-(27) did not exhibit sufficient viscosity and/or pH. Further, personal care compositions (20) and (21) were not stable enough to be evaluated under each of the accelerated aging conditions; and thus, did not pass the stability study.

### Example 4

The sensory properties of the personal care compositions (1) and (2) prepared in Example 1 were evaluated. It should be appreciated that the personal care composition (1) was the current conventional personal care composition and the personal care composition (2) was an exemplary personal care composition prepared according to the implementations disclosed herein. The sensory properties evaluated included foam volume, stringiness, structure, and fragrance release.

Foam volume was evaluated with the SITA FoamTester R-200, commercially available from SITA Lab Solutions of Dresden, Germany. Each of the personal care compositions (1) and (2) was prepared as a 5% solution with deionized water for evaluation with the SITA FoamTester R-200.

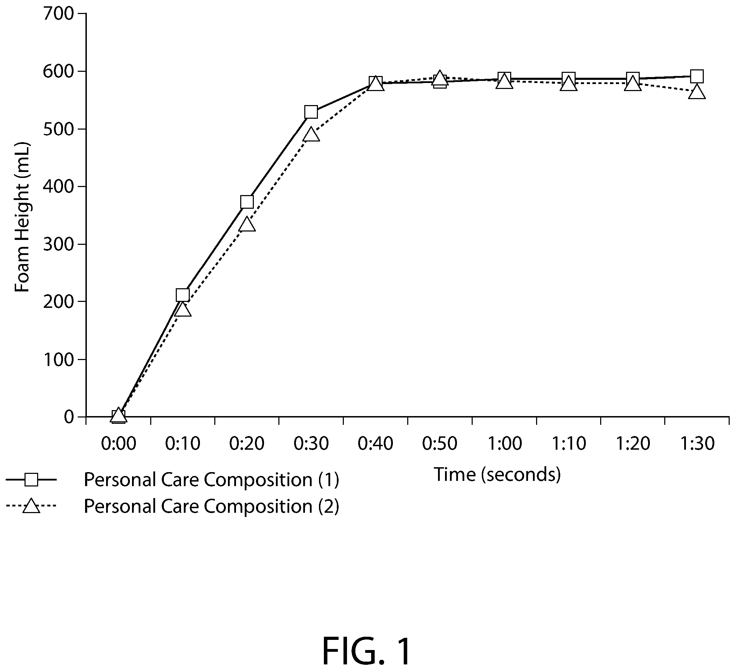

The result of the foam volume are illustrated in Figure 1 and summarized in Table 5. The time intervals or readings were made over a period of one minute and 20 seconds. As illustrated in Figure 1, the exemplary personal care composition (2) exhibited foam volume that was comparable or parity with respect to the personal care composition (1), the current conventional personal care composition. This was both surprising and unexpected as the personal care composition (1) included a relatively greater amount of surfactants and actives (i.e., surfactants and thickener) as compared to the personal care composition (2). It would be expected that the personal care composition (1) would exhibit increased foam volume based on the increased amount of the surfactants or actives present; however, the personal care composition (2), which included about 25% less actives and surfactants than the personal care composition (1) exhibited comparable foam volume.

**Table 5**

| Foam Volume Height (mL) | | |
|---|---|---|
| Time Interval | (1) | (2) |
| 1 | 0 | 0 |
| 2 | 210 | 192 |
| 3 | 373 | 338 |
| 4 | 534 | 499 |
| 5 | 581 | 582 |
| 6 | 584 | 593 |
| 7 | 586 | 587 |
| 8 | 588 | 582 |
| 9 | 588 | 581 |
| 10 | 590 | 568 |

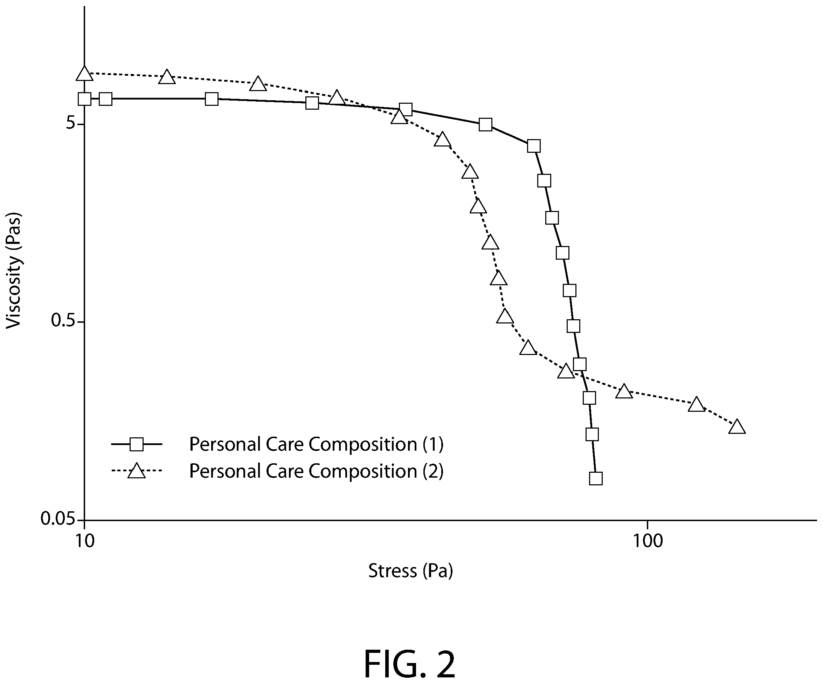

Stringiness was evaluated via rheological experiments and observing relaxation time (tₘ). The results of the stringiness study are illustrated in Figure 2. As illustrated in Figure 2, the personal care composition (2) exhibited a different rheology profile than the personal care composition (1). Specifically, the personal care composition (2) exhibited less stringiness as compared to the personal care composition (1). The reduced stringiness is best illustrated by the increased viscosity of the personal care composition (2) at higher stress, as illustrated in Figure 2.

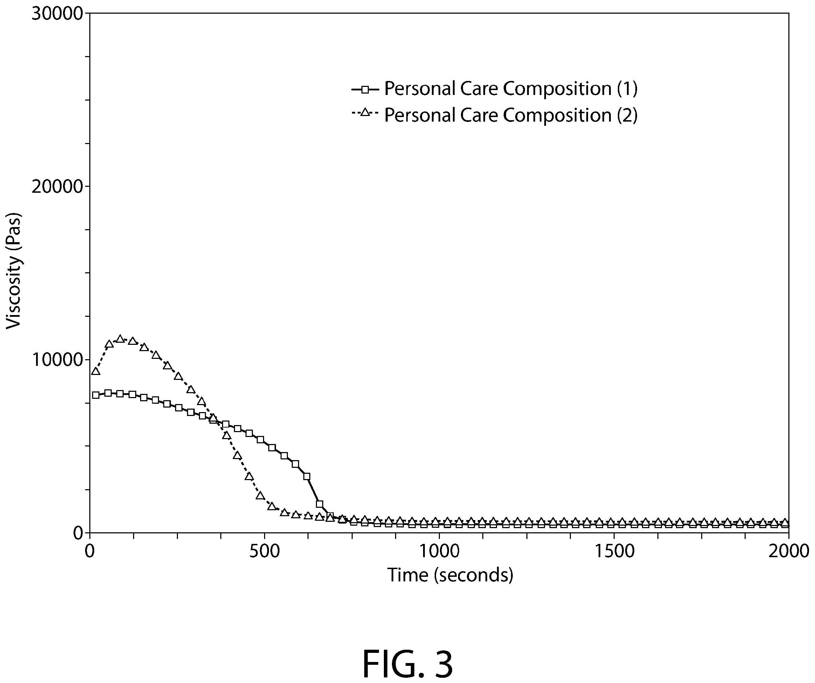

Structure of each of the personal care compositions (1)(2) was evaluated via rheological experiments to determine the rheology profile, which included evaluating viscoelastic parameters, structural properties, and temperature dependence of each of the compositions. The viscoelastic parameters characterized micellar shape, size, and density. The results of the structural study are illustrated in Figure 3. As illustrated in Figure 3, the exemplary personal care composition (2) exhibited a structural profile relatively greater than the personal care composition (1), the current conventional personal care composition. This was both surprising and unexpected as the personal care composition (1) included a relatively greater amount of surfactants and actives (i.e., surfactants and thickener) as compared to the personal care composition (2). It would be expected that the personal care composition (1) would exhibit increased structure based on the increased amount of the surfactants and actives present; however, the personal care composition (2), which included about 25% less actives and surfactants than the personal care composition (1) exhibited increased structure.

Fragrance release was evaluated via direct GC-FID analysis of headspace. Each of the personal care compositions (1) and (2) were evaluated as a neat composition as well as a 3% dilution of the respective composition. The personal care composition (2) exhibited relatively greater fragrance release as compared to the personal care composition (1). Specifically, the neat personal care composition (2) exhibited about a 19.3% increase in fragrance release as compared to the neat personal care composition (1). Further, the dilute personal care composition (2) exhibited about a 18.6% increase in fragrance release as compared to the dilute solution of the personal care composition (1).

The present disclosure has been described with reference to exemplary implementations. Although a limited number of implementations have been shown and described, it will be appreciated by those skilled in the art that changes may be made in these implementations within the scope of the claims.

## Claims

1. A personal care composition, comprising:
a surfactant system comprising at least one anionic surfactant and at least one zwitterionic surfactant,
wherein the at least one anionic surfactant comprises sodium laureth sulfate present in an amount from 4 wt% to less than 7.8 wt%,
wherein the at least one zwitterionic surfactant comprises one or more of cocamidopropyl betaine and lauryl hydroxysultaine, wherein the cocamidopropyl betaine, if present, is present in an amount of from 0.5 wt% to 2.0 wt%, and wherein the lauryl hydroxysultaine, if present, is present in an amount of from 0.2 wt% to 1.0 wt%; and
a thickener comprising esters of polyalkoxylated polyols and fatty acids,
wherein the surfactant system and the thickener are present in an amount of less than 10 wt%, wherein the thickener is present in an amount of from 0.08 wt% to 0.12 wt%, based on the total weight of the personal care composition; and
wherein the personal care composition comprises less than 0.05 weight% polyquaternium.

2. The personal care composition of claim 1, wherein the anionic surfactant further comprises one or more of ammonium lauryl ether sulfate, sodium lauryl sulfate, ammonium lauryl sulfate, triethanolamine lauryl sulfate, disodium laureth sulfosuccinate, sodium cocoyl isethionate, sodium C12-C14 olefin sulfonate, sodium laureth-6 carboxylate, sodium C12-C15 pareth sulfate, sodium methyl cocoyl taurate, sodium dodecylbenzene sulfonate, sodium cocoyl sarcosinate, triethanolamine monolauryl phosphate, or combinations thereof, and/or wherein the zwitterionic surfactant comprises one or more of a further betaine, a further sultaine, an amino propionate, or combinations thereof, optionally wherein the further betaine comprises one or more of imidazoline-based betaines, alkyl dimethyl aminoacetic acid betaines, sulfobetaines, or combinations thereof.

3. The personal care composition of claim 1 or claim 2, wherein the zwitterionic surfactant comprises at least one betaine and excludes sultaines.

4. The personal care composition of any one of the foregoing claims, wherein the surfactant system is present in an amount of from 6.8 wt% to 8.5 wt%, more preferably 7 wt% to 7.5 wt%, based on the total weight of the personal care composition.

5. The personal care composition of claim 1,
wherein the sodium laureth sulfate is present in an amount from 4.5 wt% to 6.5 wt%, preferably, 5 wt% to 5 wt%, more preferably, about 5.6 wt%, based on the total weight of the personal care composition,
wherein the cocamidopropyl betaine, if present, is present in an amount of from 0.6 wt% to 1.8 wt%, 0.8 wt% to 1.6 wt%, preferably 1.0 wt% to 1.4 wt%, or more preferably about 1.2 wt%, and
wherein the lauryl hydroxysultaine, if present, is present in an amount of from preferably 0.3 wt% to 0.9 wt%, more preferably 0.4 wt% to 0.8 wt%, even more preferably 0.5 wt% to 0.7 wt%, or about 0.6 wt%, based on the total weight of the personal care composition.

6. The personal care composition of any of the foregoing claims, wherein the personal care composition is free of cocomonoethanolamide (CMEA).

7. The personal care composition of any of the foregoing claims, wherein the thickener comprises a tetra ester made of fatty acids and a polyoxyethylene pentaerythritol having four hydrophilic poly-(ethylene glycol) arms, preferably a PEG-150 pentaerythrityl tetrastearate having four hydrophilic poly-(ethelene glycol) arms capped with stearic fatty acids, and/or wherein the thickener is present in an amount of from 0.09 wt% to 0.11 wt%, more preferably, about 0.10 wt%, based on the total weight of the personal care composition.

8. The personal care composition of any of the foregoing claims, wherein the surfactant system and the thickener are present in an amount of from 6.8 wt% to less than 10 wt%, preferably 6.8 wt% to 8.5 wt%, more preferably 7 wt% to 7.5 wt%, based on the total weight of the personal care composition.

9. The personal care composition of any of claims 1 to 8, wherein a weight ratio of the anionic surfactant to the thickener is from 45:1 to 70:1, 50:1 to 65:1, preferably 55:1 to 60:1, more preferably 56:1 to 57:1.

10. The personal care composition of any of claims 2 to 9, wherein a weight ratio of the betaine to the thickener is from 9.5:1 to 16:1, more preferably 10:1 to 15:1, even more preferably 12:1 to 14:1, even more preferably about 12:1.

11. The personal care composition of any of the foregoing claims, further comprising a salt present in an amount of 1.25 wt% or less, based on the total weight of the personal care composition, and/or comprising a viscosity of from 4,000 mPa·s (cP) to 7,500 mPa·s (cP), 4,500 mPa·s (cP) to 7,000 mPa·s (cP), or 5,000 mPa·s (cP) to 6,500 mPa·s (cP), at about 25°C, and/or comprising a pH of from 4.0 to 6.5, 4.5 to 6, more preferably, 4.5 to 5.4, 4.8 to 5.4, or about 5.

12. A method for preparing the personal care composition of any of the foregoing claims, the method comprising contacting the surfactant system and the thickener with one another.

## Patentansprüche

1. Körperpflegezusammensetzung, umfassend:
ein Tensidsystem, umfassend mindestens ein anionisches Tensid und mindestens ein zwitterionisches Tensid,
wobei das mindestens eine anionische Tensid Natriumlaurethsulfat in einer Menge von 4 Gew.-% bis weniger als 7,8 Gew.-% umfasst,
wobei das mindestens eine zwitterionische Tensid eines oder mehrere von Cocamidopropylbetain und Laurylhydroxysultain umfasst, wobei das Cocamidopropylbetain, falls vorhanden, in einer Menge von 0,5 Gew.-% bis 2,0 Gew.-% vorhanden ist, und wobei das Laurylhydroxysultain, falls vorhanden, in einer Menge von 0,2 Gew.-% bis 1,0 Gew.-% vorhanden ist; und
ein Verdickungsmittel, das Ester von polyalkoxylierten Polyolen und Fettsäuren umfasst,
wobei das Tensidsystem und das Verdickungsmittel in einer Menge von weniger als 10 Gew.-% vorhanden sind, wobei das Verdickungsmittel in einer Menge von 0,08 Gew.-% bis 0,12 Gew.-%, bezogen auf das Gesamtgewicht der Körperpflegezusammensetzung, vorhanden ist; und
wobei die Körperpflegezusammensetzung weniger als 0,05 Gew.-% Polyquaternium umfasst.

2. Körperpflegezusammensetzung nach Anspruch 1, wobei das anionische Tensid weiterhin eines oder mehrere der folgenden Tenside umfasst:
Ammoniumlaurylethersulfat, Natriumlaurylsulfat, Ammoniumlaurylsulfat, Triethanolaminlaurylsulfat, Dinatriumlaurethsulfosuccinat, Natriumcocoylisethionat, Natrium-C12-C14-Olefinsulfonat, Natriumlaureth-6-carboxylat, Natrium-C12-C15-parethsulfat, Natriummethylcocoyltaurat, Natriumdodecylbenzolsulfonat, Natriumcocoylsarcosinat, Triethanolaminmonolaurylphosphat oder Kombinationen davon, und/oder wobei das zwitterionische Tensid eines oder mehrere eines weiteren Betains, eines weiteren Sultains, eines Aminopropionats oder Kombinationen davon umfasst, wobei das weitere Betain wahlweise eines oder mehrere von Betainen auf Imidazolinbasis, Alkyl-Dimethylaminoessigsäurebetainen, Sulfobetainen oder Kombinationen davon umfasst.

3. Körperpflegezusammensetzung nach Anspruch 1 oder Anspruch 2, wobei das zwitterionische Tensid mindestens ein Betain umfasst und Sultaine ausschließt.

4. Körperpflegezusammensetzung nach einem beliebigen der vorhergehenden Ansprüche, wobei das Tensidsystem in einer Menge von 6,8 Gew.-% bis 8,5 Gew.-%, vorzugsweise 7 Gew.-% bis 7,5 Gew.-%, bezogen auf das Gesamtgewicht der Körperpflegezusammensetzung, vorhanden ist.

5. Körperpflegezusammensetzung nach Anspruch 1,
wobei das Natriumlaurethsulfat in einer Menge von 4,5 Gew.-% bis 6,5 Gew.- %, vorzugsweise 5 Gew.-% bis 5 Gew.-%, noch bevorzugter etwa 5,6 Gew.-%, bezogen auf das Gesamtgewicht der Körperpflegezusammensetzung, vorhanden ist,
wobei das Cocamidopropylbetain, falls vorhanden, in einer Menge von 0,6 Gew.-% bis 1,8 Gew.-%, 0,8 Gew.-% bis 1,6 Gew.-%, vorzugsweise 1,0 Gew.-% bis 1,4 Gew.-% oder noch bevorzugter etwa 1,2 Gew.-% vorhanden ist, und
wobei das Laurylhydroxysultain, falls vorhanden, in einer Menge von vorzugsweise 0,3 Gew.-% bis 0,9 Gew.-%, noch bevorzugter 0,4 Gew.-% bis 0,8 Gew.-%, noch mehr bevorzugt 0,5 Gew.-% bis 0,7 Gew.-% oder etwa 0,6 Gew.-%, bezogen auf das Gesamtgewicht der Körperpflegezusammensetzung, vorhanden ist.

6. Körperpflegezusammensetzung nach einem beliebigen der vorhergehenden Ansprüche, wobei die Körperpflegezusammensetzung frei von Cocomonoethanolamid (CMEA) ist.

7. Körperpflegezusammensetzung nach einem beliebigen der vorhergehenden Ansprüche, wobei das Verdickungsmittel einen Tetraester aus Fettsäuren und einem Polyoxyethylenpentaerythrit mit vier hydrophilen Poly(ethylenglycol)-Armen umfasst, vorzugsweise ein PEG-150-Pentaerythrityltetrastearat mit vier hydrophilen Poly(ethylenglycol)-Armen, die mit Stearinsäuren gekappt sind, und/oder wobei das Verdickungsmittel in einer Menge von 0,09 Gew.-% bis 0,11 Gew.-%, vorzugsweise etwa 0,10 Gew.-%, bezogen auf das Gesamtgewicht der Körperpflegezusammensetzung, vorhanden ist.

8. Körperpflegezusammensetzung nach einem beliebigen der vorhergehenden Ansprüche, wobei das Tensidsystem und das Verdickungsmittel in einer Menge von 6,8 Gew.-% bis weniger als 10 Gew.-%, vorzugsweise 6,8 Gew.-% bis 8,5 Gew.-%, noch bevorzugter 7 Gew.-% bis 7,5 Gew.-%, bezogen auf das Gesamtgewicht der Körperpflegezusammensetzung, vorliegen.

9. Körperpflegezusammensetzung nach einem beliebigen der Ansprüche 1 bis 8, wobei das Gewichtsverhältnis des anionischen Tensids zum Verdickungsmittel 45:1 bis 70:1, 50:1 bis 65:1, vorzugsweise 55:1 bis 60:1, noch bevorzugter 56:1 bis 57:1 beträgt.

10. Körperpflegezusammensetzung nach einem beliebigen der Ansprüche 2 bis 9, wobei das Gewichtsverhältnis von Betain zu Verdickungsmittel 9,5:1 bis 16:1, vorzugsweise 10:1 bis 15:1, noch bevorzugter 12:1 bis 14:1, noch bevorzugter etwa 12:1 beträgt.

11. Körperpflegezusammensetzung nach einem beliebigen der vorhergehenden Ansprüche, die weiterhin ein Salz in einer Menge von 1,25 Gew.-% oder weniger, bezogen auf das Gesamtgewicht der Körperpflegezusammensetzung, und/oder eine Viskosität von 4.000 mPa·s (cP) bis 7.500 mPa·s (cP), 4.500 mPa·s (cP) bis 7.000 mPa·s (cP) oder 5.000 mPa·s (cP) bis 6.500 mPa·s (cP) bei etwa 25 ° aufweist, und/oder einen pH-Wert von 4,0 bis 6,5, 4,5 bis 6, vorzugsweise 4,5 bis 5,4, 4,8 bis 5,4 oder etwa 5 aufweist.

12. Verfahren zur Herstellung der Körperpflegezusammensetzung nach einem beliebigen der vorhergehenden Ansprüche, wobei das Verfahren das Inkontaktbringen des Tensidsystems und des Verdickungsmittels miteinander umfasst.

## Revendications

1. Composition de soins personnels, comprenant :
un système tensioactif comprenant au moins un tensioactif anionique et au moins un tensioactif zwitterionique,
dans laquelle l'au moins un tensioactif anionique comprend du laureth sulfate de sodium présent en une quantité de 4 % en poids à moins de 7,8 % en poids,
dans laquelle l'au moins un tensioactif zwitterionique comprend un ou plusieurs tensioactifs parmi la cocamidopropyl bétaïne et la lauryl hydroxysultaine, dans laquelle la cocamidopropyl bétaïne, si elle est présente, est présente en une quantité de 0,5 % en poids à 2,0 % en poids, et dans laquelle la lauryl hydroxysultaine, si elle est présente, est présente en une quantité de 0,2 % en poids à 1,0 % en poids ; et
un épaississant comprenant des esters de polyols polyalcoxylés et des acides gras,
dans laquelle le système tensioactif et l'épaississant sont présents en une quantité inférieure à 10 % en poids, dans laquelle l'épaississant est présent en une quantité de 0,08 % en poids à 0,12 % en poids, par rapport au poids total de la composition de soins personnels ; et
dans laquelle la composition de soins personnels comprend moins de 0,05 % en poids de polyquaternium.

2. Composition de soins personnels selon la revendication 1, dans laquelle le tensioactif anionique comprend en outre un ou plusieurs tensioactifs parmi le lauryl éther sulfate d'ammonium, le lauryl sulfate de sodium, le lauryl sulfate d'ammonium, le lauryl sulfate de triéthanolamine, le laureth sulfosuccinate disodique, le cocoyl iséthionate de sodium, l'oléfine sulfonate de sodium en C12-C14, le laureth-6 carboxylate de sodium, le pareth sulfate de sodium en C12-C15, le méthyl cocoyl taurate de sodium, le dodécyl benzène sulfonate de sodium, le cocoyl sarcosinate de sodium, le monolauryl phosphate de triéthanolamine, ou les combinaisons de ceux-ci, et/ou dans laquelle le tensioactif zwitterionique comprend un ou plusieurs tensioactifs parmi une bétaïne supplémentaire, une sultaine supplémentaire, un aminopropionate, ou les combinaisons de ceux-ci, éventuellement dans laquelle la bétaïne supplémentaire comprend une ou plusieurs bétaïnes parmi les bétaïnes à base d'imidazoline, les acide alkyl diméthyl aminoacétique bétaïnes, les sulfobétaïnes, ou les combinaisons de celles-ci.

3. Composition de soins personnels selon la revendication 1 ou la revendication 2, dans laquelle le tensioactif zwitterionique comprend au moins une bétaïne et exclut les sultaïnes.

4. Composition de soins personnels selon l'une quelconque des revendications précédentes, dans laquelle le système tensioactif est présent en une quantité de 6,8 % en poids à 8,5 % en poids, de préférence de 7 % en poids à 7,5 % en poids, par rapport au poids total de la composition de soins personnels.

5. Composition de soins personnels selon la revendication 1,
dans laquelle le laureth sulfate de sodium est présent en une quantité de 4,5 % en poids à 6,5 % en poids, de préférence, de 5 % en poids à 5 % en poids, et plus préférablement, d'environ 5,6 % en poids, par rapport au poids total de la composition de soins personnels,
dans laquelle la cocamidopropyl bétaïne, si elle est présente, est présente en une quantité de 0,6 % en poids à 1,8 % en poids, de 0,8 % en poids à 1,6 % en poids, de préférence de 1,0 % en poids à 1,4 % en poids, ou plus préférablement d'environ 1,2 % en poids, et
dans laquelle la lauryl hydroxysultaine, si elle est présente, est présente en une quantité de préférence de 0,3 % en poids à 0,9 % en poids, plus préférablement de 0,4 % en poids à 0,8 % en poids, et encore plus préférablement de 0,5 % en poids à 0,7 % en poids, ou d'environ 0,6 % en poids, par rapport au poids total de la composition de soins personnels.

6. Composition de soins personnels selon l'une quelconque des revendications précédentes, dans laquelle la composition de soins personnels est exempte de cocomonoéthanolamide (CMEA).

7. Composition de soins personnels selon l'une quelconque des revendications précédentes, dans laquelle l'épaississant comprend un tétraester constitué d'acides gras et d'un pentaérythritol polyoxyéthyléné comportant quatre chaînes hydrophiles de poly(éthylène glycol), de préférence un tétrastéarate de pentaérythrityle PEG-150 comportant quatre chaînes hydrophiles de poly(éthylène glycol) coiffées par des acides gras stéariques, et/ou dans laquelle l'épaississant est présent en une quantité de 0,09 % en poids à 0,11 % en poids, plus préférablement, d'environ 0,10 % en poids, par rapport au poids total de la composition de soins personnels.

8. Composition de soins personnels selon l'une quelconque des revendications précédentes, dans laquelle le système tensioactif et l'épaississant sont présents en une quantité de 6,8 % en poids à moins de 10 % en poids, de préférence de 6,8 % en poids à 8,5 % en poids, et plus préférablement de 7 % en poids à 7,5 % en poids, par rapport au poids total de la composition de soins personnels.

9. Composition de soins personnels selon l'une quelconque des revendications 1 à 8, dans laquelle le rapport pondéral du tensioactif anionique à l'épaississant est compris entre 45:1 et 70:1, entre 50:1 et 65:1, de préférence entre 55:1 et 60:1, et plus préférablement entre 56:1 et 57:1.

10. Composition de soins personnels selon l'une quelconque des revendications 2 à 9, dans laquelle le rapport pondéral de la bétaïne à l'épaississant est compris entre 9,5:1 et 16:1, plus préférablement entre 10:1 et 15:1, encore plus préférablement entre 12:1 et 14:1, encore plus préférablement autour de 12:1.

11. Composition de soins personnels selon l'une quelconque des revendications précédentes, comprenant en outre un sel présent en une quantité de 1,25 % en poids ou moins, par rapport au poids total de la composition de soins personnels, et/ou présentant une viscosité de 4 000 mPa·s (cP) à 7 500 mPa·s (cP), de 4 500 mPa·s (cP) à 7 000 mPa·s (cP), ou de 5 000 mPa·s (cP) à 6 500 mPa·s (cP), à environ 25 °C, et/ou présentant un pH de 4,0 à 6,5, de 4,5 à 6, plus préférablement, de 4,5 à 5,4, de 4,8 à 5,4, ou d'environ 5.

12. Procédé de préparation de la composition de soins personnels selon l'une quelconque des revendications précédentes, le procédé comprenant la mise en contact du système tensioactif et de l'épaississant l'un avec l'autre.
